# EUROPEAN PATENT SPECIFICATION

(11) **EP 1 664 486 B1**
(45) Date of publication and mention of the grant of the patent: **27.02.2013**
(21) Application number: 04770773.2
(22) Date of filing: 02.09.2004
(51) Int. Cl.: E02D 1/02, E21F 17/00, G01N 33/24

(54) **APPARATUS FOR MONITORING GEOTECHNICAL AND STRUCTURAL PARAMETERS OF SOILS, ROCKS AND STRUCTURES IN GENERAL**
VORRICHTUNG ZUR ÜBERWACHUNG VON GEOTECHNISCHEN UND STRUKTURELLEN PARAMETERN VON BÖDEN, GESTEIN UND STRUKTUREN ALLGEMEIN
APPAREIL POUR CONTROLER DES PARAMETRES GEOTECHNIQUES ET STRUCTURELS DE SOLS, ROCHES ET STRUCTURES EN GENERAL

(30) Priority: 09.09.2003 IT SV20030034
(43) Date of publication of application: 07.06.2006
(73) Proprietor: C.S.G. S.R.L., 15010 Ricaldone (AL) (IT)
(72) Inventor: FOGLINO, Luigi, I-15010 Ricaldone (IT); FOGLINO, Daniela, I-15010 Ricaldone (IT); LOVISOLO, Mario, I-15010 Ricaldone (IT)
(74) Representative: Karaghiosoff, Giorgio Alessandro
(86) International application number: PCT/IT2004/000474
(87) International publication number: WO 2005/024415

(56) References cited:
- EP-A- 0 505 276
- US-A- 5 337 613
- US-A- 5 475 187
- US-A- 5 661 464

## Description

The invention relates to an apparatus for monitoring geotechnical and structural parameters of soils, rocks and structures in general, according to the preamble of claim 1.

Such apparatuses are known to find application in stability diagnosis, for instance on natural and artificial slopes and excavations, in dam and tunnel monitoring, in the civil engineering works foundation soil investigation, as well as in other fields. These apparatuses are typically introduced in the soil to be monitored, i.e. in specially drilled geognostic boreholes, and secured to the soil, rock or structure to be monitored by grouting or the like. This locking method has the drawback of often providing a poor adhesion of the apparatus to the borehole walls, an imperfect water tightness in groundwater separation, particularly for deep installations, and finally rather long execution times.

A further shortcoming of prior art apparatuses is that they are constructed in such a manner as to include a single sensor for a single parameter, whereby all-parameter monitoring may be only achieved by using several single parameter dedicated apparatuses. Thus, a set of signals from different sensors are obtained, which are not strictly related to each other, and whose signal format structure may cause compatibility problems for transmission by common transmission means. Therefore, dedicated interfaces must be provided between individual apparatuses and transmission units and between individual apparatuses and processor units.

Such an apparatus is described for instance by document US5337613A, which describes a measuring instrument including first and second rigid, elongate measuring elements which are interconnected by a flexible element. The measuring elements are supported in a measuring tube inserted in the foundation soil. Whenever a displacement occurs in the foundation soil or construction, the measuring tube is deformed and the second measuring element is pivoted with respect to the first measuring element. The curvature or bending which thereby occurs in the flexible element of suitable dimensioning or configuration is preferably determined by at least one wire strain gage fitted to the flexible element in the plane of deformation, the elongations being converted, for example by use of a Wheatstone bridge circuit, into proportional electrical current or voltage signals. Preferably, wire strain gages are disposed on all four mutually perpendicular outer sides of the flexible element, in order to allow for the detection of displacements in mutually perpendicular planes.

Document US5475187A also describes a string of sondes designed to be lowered down a borehole by means of a cable in order to detect the arrival of seismic waves therein, the sondes including retractable anchor means that are hydraulically actuateable and that are connected by a hydraulic circuit to a common source of hydraulic pressure. The string includes means for putting said hydraulic circuit to ambient pressure, said means being connected to said cable and being adapted to act on said hydraulic circuit in order to cause said anchor means to be retracted when a traction force exceeding a selected value is exerted on said cable.

Document US5661464A further describes an apparatus comprising a probe for measuring snow pack movement, comprising a mobile element adapted to contact and be displaced by the snow pack, a static element linked to the mobile element by linkage means and adapted to remain stationary relative to the ground, and measuring means to measure relative movement of the two elements. The mobile element comprises a wand adapted to be placed in contact with a snowpack and having tilt means that permit the wand to tilt away from the vertical in response to lateral movement of the surrounding snow pack. The measuring means comprises a movement sensor connected to the wand that measures the resulting movement of the wand. Data relay means transmits electronic signals generated by the movement sensor to a remote data collection and storage means. The invention further comprises a method for measuring the condition of an avalanche-prone snow pack, employing one or more the probes, linked to a central data collecting and transmitting means, and transmitting the resulting information to a remote data measuring station.

Finally, also document EP0505276A describes a probe which consists of a string of essentially tubular cartridges connected together by flexible links, at least some of the said cartridges accommodating means for measuring microseismic quantities representative of the acoustic emission induced by the injection of a fluid into the well, characterised in that all the cartridges have a diameter which is small enough for them to be inserted into a pipe column installed in the well. At the same time, a measurement method is proposed which makes use of this probe during an injection test comprising at least one fluid injection period followed by a rest period, the well pipe column comprising at least one level of perforations, characterised in that the probe is lowered into the well and is then immobilised near the level of perforations so as to measure at least the microseismic quantities preferably associated with hydraulic quantities during at least the rest period.

Thus, the object of this invention is to overcome the above drawbacks and provide, by simple and inexpensive means, an apparatus as described hereinbefore, which affords a considerable functional improvement as compared with prior art apparatuses, allows easier and accurate monitoring of the relevant soil or structure, while allowing to collect a plurality of different data by using a single apparatus and by drilling a single borehole, unlike prior art. A further object is to provide an apparatus that is easily adaptable to different monitoring situations, particularly to landslide soil displacements, while providing a more accurate data detection and collection. It is further desirable to allow the apparatus to be perfectly integrated with the geognostic borehole wall in a simple, fast and possibly reversible manner, to allow an easy removal of the probe, and repositioning and/or reuse thereof in another borehole.

The invention fulfils the above objects by providing an apparatus as described hereinbefore, further comprising the features of the characterizing part of claim 1.

Thanks to this arrangement, the apparatus may be even adapted to a borehole having an imperfect straightness, caused by landslide displacements occurring when the borehole has been already drilled and the apparatus has been introduced therein.

The enclosures and/or the non-rigid connecting members may be of the closed type, and be impervious at least to external liquids, to keep any infiltration from damaging the equipment contained in each enclosure and/or the connection members.

The enclosures may be arranged alternate with the connection members.

Advantageously, the connection members between the enclosures may be elastic, to restore the initial mutual enclosure positioning condition, with reference to the at least one degree of freedom of motion or to all the degrees of freedom of motion of the enclosures relative to each other.

The non-rigid connection members may include elastic means for restoring the initial idle or unstressed condition of the apparatus.

According to a preferred embodiment of the invention, the enclosures may have a tubular, preferably cylindrical shape and particularly have equal diameters, and equal or different axial extensions.

The connection members may also have a substantially tubular shape, and be made, for instance, of steel braided rubber, or extend along tubular, preferably cylindrical surfaces, and particularly have substantially the same diameter as the tubular enclosures.

The connection members may be stabilized in the pre-holes or in monitoring pipes by annular centralizers secured between the flexible connecting joints and the enclosures, whose outside diameter is equal to the diameter of the pre-hole or to the inside diameter of the monitoring pipe.

The connection members may be helical springs, preferably made of steel, whose elastic properties are adapted to the characteristics of the whole apparatus, which are arranged coaxially to the tubular enclosures.

Each helical spring may be fastened at its two opposite ends to the corresponding ends of two adjacent enclosures.

Each helical spring may be outwardly hermetically covered by at least one flexible sleeve or cuff, particularly bellows-shaped, preferably made of plastics, rubber or the like and having an appropriate thickness, possibly reinforced with a steel braid, to prevent infiltrations, e.g. of mud or moisture, in the connection member.

At least two adjacent enclosures may be connected by a tie having such a length as to maintain the helical spring in an intermediate pressure loading condition. This arrangement prevents any excessive extension of the spring, which might involve the risk of distortion thereof, and possibly provides an automatic elastic adaptation, within certain limits, of the apparatus' length to any increased length of the borehole, caused by soil or structure displacements.

At least one of the ends of the tie may be dynamically connected to a sensor which detects the relative position of the two enclosures, and is accommodated in one of the two enclosures. The opposite end is fixedly anchored to the adjacent enclosure.

This sensor may be a permanent magnet which is capable of sliding in both directions toward the tie, which magnet cooperates with an induction sensor for determining magnet displacement by magnetic induction. The sensor may be equipped with elastic means for pulling the magnet toward compression of the immediately adjacent connecting spring.

The tie may be of such a length as to keep the magnet of the associated enclosure axial position sensor in an intermediate signaling position when the apparatus is in an idle or unstressed position, so as to be able to measure any relative movement of the two enclosures, toward and away from each other.

The tie may be a wire or a rod, particularly sheathed, and extend substantially along the central axis of the apparatus.

Eventually, the enclosures, the connection members and the connecting ties between the enclosures may form an assembly of members to transfer soil displacement stresses to position, pressure, inclination and acceleration sensors or transducers or on further sensors for monitoring other particular parameters.

The enclosures may have closing members at their ends, which may be inserted therein and secured thereto. These members act as terminals allowing attachment and/or passage of connecting springs, sealing sleeves, ties and one or more position sensors.

Alternatively, at each opposite end of each enclosure, a substantially tubular rigid terminal may be provided for coupling each end of the enclosure to the corresponding ends of the two immediately adjacent springs. The interposition of this rigid terminal provides advantages as detailed below.

In accordance with an advantageous improvement, at least some of the connection members may have axial extension limiting means, for preventing the spring extension range from being exceeded, and the spring from being consequently permanently deformed or broken. As mentioned above, this function is at least partly accomplished by the above tie, but this invention has the object of providing means to withstand even considerable axial stresses caused by particularly wide landslide displacements.

These extension stop means may be one or more inextensible elongate members, each extending inside or outside each spring, parallel to the axis thereof. One of the opposite ends of an inextensible member may have a widened shape and be slideably accommodated in a chamber which has two opposite extension and compression stop abutments. This chamber may be provided within the closing member at the end of an adjacent enclosure or within the associated substantially tubular rigid coupling terminal, whereas the opposite end of the inextensible member may be secured at the closing member or at the rigid member for coupling the spring to the other enclosure.

Nevertheless, according to a preferred embodiment of the invention, to be further detailed in the description of the drawings, each of the opposite ends of each inextensible member may have a widened shape and be slideably accommodated inside a respective chamber having two opposite extension and compression stop abutments, each of said chambers being provided within each opposite closing member at each end of each enclosure or within the substantially tubular rigid coupling terminals. Thanks to this arrangement, both ends of each inextensible member may slide within their respective chambers, thereby distributing the retaining action to both ends of the spring and preventing an excessive displacement of said ends. Moreover, thanks to the above arrangements, each inextensible member may also possibly accomplish the function of an axial compression stop means for the spring. In fact, as soon as an excessive compression of the spring is attempted, the widened end of the inextensible member may abut to stop the compression motion and keep the spring from being actually excessively compressed and the whole apparatus from being damaged. In this case, the inextensible members may be advantageously flexible but relatively rigid, and be thus prevented from sensibly bowing outwardly and/or inwardly.

Typically, each of these inextensible stop members may be a wire or a rod, preferably made of harmonic steel. The inextensible members may be arranged along the outer circumference of the spring.

When the apparatus is in the idle or unstressed condition, the widened ends of each inextensible member may be in a spring compression stop abutment position, so that a longer stroke is available for spring extension.

Thanks to the provision of the inextensible members, the apparatus may be kept from being broken, e.g. due to landslide displacements. It should be noted that, when the apparatus is broken, the portion thereof that remains in the borehole would be hardly recoverable, and this would have a considerable impact on costs, due to the high cost of the sensors contained in the enclosures.

According to a further important improvement, means may be provided for removably securing at least a portion of the apparatus to the soil, rock or structure to be monitored, whereby grouting may be avoided, as well as the consequent drawbacks described above.

These securing means may be one or more anchor sections, which are arranged in a predetermined order relative to the enclosures and to the connecting springs.

Advantageously, these anchor sections may be of the closed type and be impervious to at least external liquids.

The locking action may be obtained by radial expansion of the anchor sections within the hole in which the apparatus is housed.

In accordance with a preferred embodiment, to be further detailed in the description of the drawings, one locking section may be provided respectively immediately upstream and downstream from each enclosure. Therefore, each of these sections is interposed between an end of the enclosure and the substantially tubular rigid terminal for connection to the immediately adjacent spring.

Each locking section may have a substantially tubular cylindrical shape, with substantially the same outside diameter as the enclosures, and be formed by an inner cylindrical wall and an outer cylindrical wall which are arranged coaxially and define a space therebetween. At least one of the two walls, preferably the outer wall, may be made of an extensible or expansible material, such as rubber or the like.

The locking action may be achieved by an outward radial expansion of the outer wall of each anchor section, which is obtained by introducing a pressurized fluid, particularly air, water or oil, in the empty space between the two walls of the section.

At least one conduit, particularly a single continuous conduit may be provided for feeding pressurized fluid to the anchor sections, which is connected at its outside end to a pressurized fluid source, and extends within each anchor section, parallel to the axis thereof. This conduit may have one or more radial apertures, which communicate with the empty space between the two walls.

In accordance with a preferred embodiment, the feeding conduit may be formed by perforating the thickness of the inner non expandable tubular wall of each anchor section.

Level with each enclosure, this feeding conduit may extend therein, parallel to the axis thereof, or be obtained by perforation of the thickness of the enclosure wall.

Level with the helical connecting springs, the conduit may be formed by a portion of flexible tube, particularly spiraled with a smaller radius than the springs themselves, to be able to accommodate spring deformations with no possible interference therewith. Therefore, the anchor members are connected in series by a single feeding conduit.

One or more enclosures may contain, particularly in an intermediate position of their axial extension, at least one member for carrying the monitoring sensor unit, which is composed of one or more sensors.

In at least some of the enclosures, inclination sensors may be provided for inclinometric measurements and/or displacement transducers and/or seismic sensors and/or temperature sensors, and/or pressure or piezometric level sensors, and/or electric potential meters and/or any other sensor required for monitoring particular parameters, for instance soil radioactivity.

An electronic card may be provided in at least one enclosure, for collecting and/or locally processing the data provided by the sensors of the corresponding enclosure.

A multicore cable may be further provided for transmitting the data collected by sensors, which extends from the last enclosure to the first enclosure of the succession of enclosures, and is successively led outside through a liquid-tight passage.

Advantageously, level with the helical connecting springs this multicore transmission cable may be spiraled with a smaller radius than the springs, to ensure a perfect fit thereof with no risk of pinching.

According to an improvement, the spring winding may contain at least one element which extends axially from one end to the other of the spring, which element is made of an elastically deformable material, to be axially compressible/extensible and deformable. At least one helical groove may be provided on the outer surface of this element, for accommodating the pressurized fluid feeding conduit and/or the multicore transmission cable. This advantageously allows an orderly arrangement of the multicore cable and the feeding conduit level with the springs, thereby avoiding any interference therebetween and/or between spring turns.

The multicore transmission cable may be discontinuous and connect the cards for collecting the data provided by the sensors associated to the enclosures. To this end, these data collection cards may have inputs and outputs for the transmission line, and a receive and transmit section based on multichannel or multitasking protocols or transmission buses. All these arrangements advantageously allow the system to provide a high electronic modularity, thanks to the fact that a variable number of cards may be provided and connected "in series", as a function of the number of enclosures of the system.

The temperature sensor and/or the pressure sensor may communicate with the outside of their respective enclosures. To this end, the sensitive portion of these sensors may be housed or communicate with an outer side recess or undercut of the enclosure, while being hermetically isolated from the inside of the enclosure.

The peripheral wall of the enclosure may have an outer wall with microslots at the recess for communication between the sensors and the outside. Particularly, the recess used by temperature and/or pressure sensors to communicate with the outside may be filled with a porous material.

A local data collection control unit may be advantageously provided, which is connected to the transmission cable and transmit controls to the data collection cards and/or the sensors, e.g. to reset a sensor, or to toggle a sensor on and off whenever data is to be detected at given time intervals.

The unit, which may be separate or integrated in a first enclosure, may further have means for radio transmission/reception of controls and data to/from a central supervision location.

Finally, the local unit may be programmable relative to the functions to be accomplished, such as sensor actuating and/or reading times, alarm thresholds or the like.

The advantages of this invention are self-evident from the above description and consist in that it allows simultaneous multiparameter monitoring, by using a single apparatus in a single borehole. The particular modular design of the apparatus allows to perform differential detections of the main physical and mechanical parameters of the soil and structures in three dimensions (angular deviation on the azimuthal plane and extension or compression on the zenithal plane), of inclination (inclinometric parameters), and extension (extensometric parameters), piezometric, acceleration (accelerometric parameters), seismic, temperature and spontaneous potential detections, all at the same time and in a single borehole, obviously as a function of the equipment contained in the apparatus. Prior art monitoring apparatus do not allow to perform simultaneous inclinometric, extensometric, piezometric detections (in the same borehole). Simultaneous and concurrent detection of these parameters is particularly convenient in stability diagnosis on natural and artificial slopes and excavations, in dam and tunnel monitoring, in the analysis of civil engineering works foundation soils. The special flexible and extensible/compressible spring-like joint allows the enclosures to fit the features of the hole, while maintaining the azimuthal directionality with respect to a preset external reference system. The apparatus further allows automatic and continuous remote detection of all the above parameters by using a single control unit. The local detection unit allows continuous detection of all parameters with a single control unit, which is preferably installed at the top of the borehole. This allows to set alarm thresholds, transmit information in real time to control locations and request civil protection interventions. The monitoring chain formed by the succession of enclosures and elastic joints is highly adaptable to the deformations caused by soil or structure displacements. The particular design of the enclosure and elastic joint assembly also provides a high adaptability to any subsoil deformation (landslide displacements), as well as a high protection of the sensors and electronic components installed therein, while ensuring an optimized soil-sensor coupling and a long life (for instance as compared with prior art inclinometer tubes). Another considerable advantage is the possibility to restore and use highly deviated boreholes, which are no longer suitable for measurements by prior art equipment. The maintenance of the so-called azimuthal direction and the high flexibility provided by the particular joint that connects sensor enclosures allow the apparatus to be free from prior art mechanical references (guides and inclinometer tubes). This allows to restore and measure damaged conventional inclinometer tubes which cannot be read by prior art inclinometer apparatuses. The device of the invention provides a very high space resolution of measurements. In fact, enclosures may have a very small axial size, particularly 20 centimeters, and allow a considerable spatial resolution improvement for the analysis of localized discontinuities, as compared with prior art stationary inclinometer apparatuses (In-place Inclinometers). The modular design of enclosures and connecting joints, the capability of monitoring within a single borehole and of continuing detections even when the monitoring chain is highly deformed allow to considerably reduce construction, installation and monitoring costs. The particular radial expansion system for securing the apparatus to its geognostic borehole adds effectiveness to the anchorage and to groundwater level separation (in case of geologically existing groundwaters) with lower execution times. The anchor sections provide a perfect attachment of the apparatus to the soil, rocks or structure to be monitored, even when groundwater is present, and prevent it from sliding and/or moving relative to the soil. Moreover, when needed, fluid may be removed from anchor sections for probe recovery and/or repositioning. Finally, the inextensible members provide a high tensile strength of the spring connecting member, and keep it from being permanently deformed and/or broken.

Further characteristics and improvements will form the subject of the dependent claims.

The characteristics of the invention and the advantages derived therefrom will be more apparent from the following detailed description of the annexed drawings, in which:
- Fig. 1 is a schematic sectional view of a first embodiment of the inventive apparatus, when installed in the soil.
- Fig. 2 shows the behavior of the apparatus of Fig. 1 after a landslide displacement.
- Figs. 3A and 3B are sectional views of a first preferred embodiment of a sensor enclosure and a connection member.
- Fig. 4 is a sectional view as taken across line A-A' of fig. 3.
- Fig. 5 is a partly exposed perspective view of a portion of a second embodiment of the apparatus according to the invention.
- FIG. 6A is a side view of the portion of the apparatus of FIG. 5.
- Fig. 6B is an enlarged view of the inclinometer/extensometer joint of Fig. 6A.
- Fig. 6C is a view of Figure 6A with the apparatus introduced in a hole drilled in the soil and with hydraulic packers dilated for securing the apparatus in said hole.
- Fig. 7 is a sectional view as taken across line B-B' of fig. 6.
- Figs. 8A to 8E are longitudinal axial sectional views of a portion of the apparatus of Fig. 5, with the locking sections in an unexpanded condition.
- Fig. 9 is a sectional view, as taken across a diametrical plane, of a third embodiment of an enclosure element of the inventive apparatus.
- Fig. 10 is a sectional view, as taken across a diametrical plane, of a third embodiment of a joint according to the invention, whose ends are fastened to the ends of successive enclosures.
- Figs. 11 to 13 show in greater detail the embodiment of the previous Figures 9 and 10.

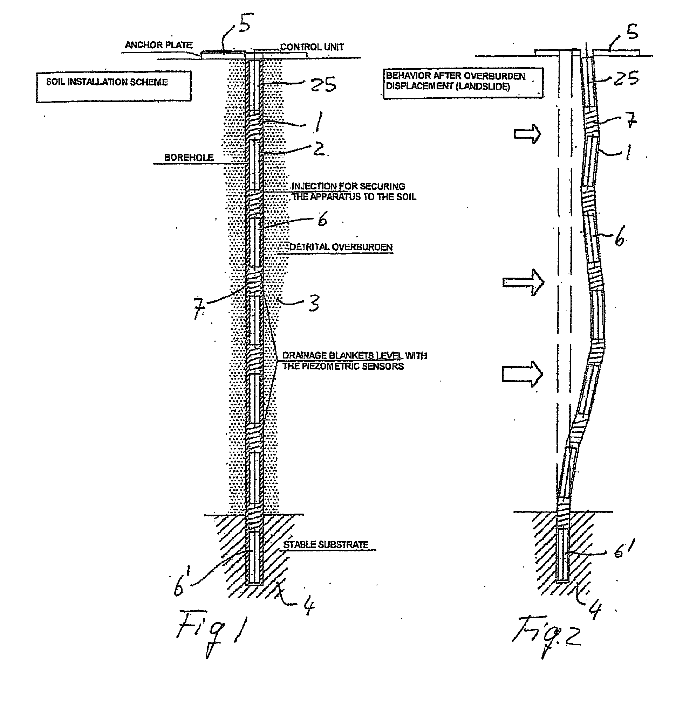

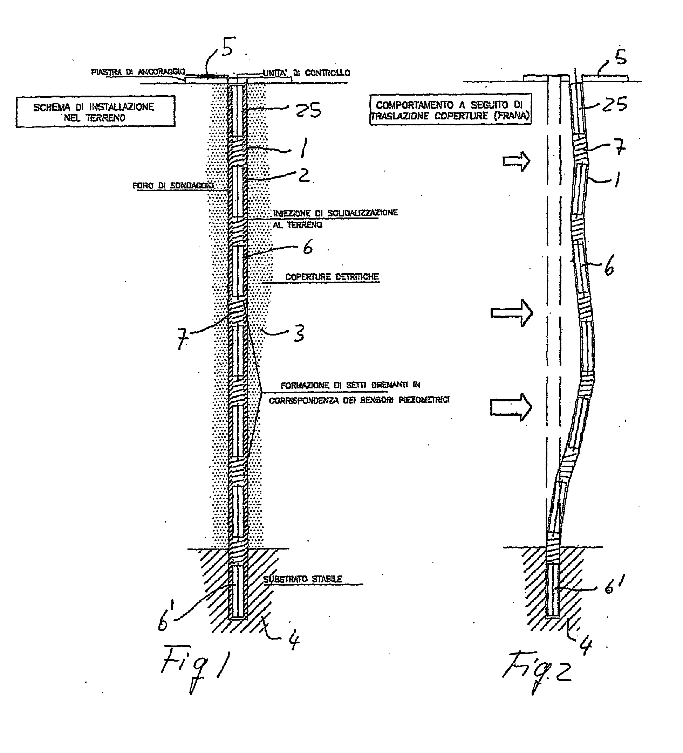

Referring to Fig. 1, the apparatus of the invention, regardless of the embodiments being used, is introduced in a borehole 1 which extends through the thickness of the soil 3 to be monitored until it reaches a stable substrate 4, and somewhat deeper therein. Landslide displacements are known to be typically caused by the sliding motion of a mass 3 over a stable substrate 4. In certain particular instances, the apparatus may have a smaller axial extension and not reach a stable substrate 4, e.g. when a surface layer of soil 3 is only to be monitored. In the particular case of the embodiment that is shown in Fig. 1, the apparatus is secured to the soil by appropriate grout and/or resin injections 2, to perfectly accommodate any displacement. Nevertheless, as previously mentioned and better explained hereafter, this invention proposes a second embodiment of the apparatus, having locking means alternative to grouting, which obviate the drawbacks associated to the latter. At its outside end, the apparatus is secured to the surface of the soil 3 by an anchor plate 5.

The apparatus is composed of a succession of tubular enclosures 6 or container modules, alternated with connection joints, formed by springs 7. Hence, these joints are flexible, extensible and compressible and allow the individual enclosures 6 to perform relative motions, and particularly allow them to move away and toward each other and to angularly offset or displace one enclosure 6 relative to another, to fit any change of the borehole 1 caused to translational soil displacements, as shown by the arrows of Fig. 2. The amount of displacement of soil 3 may vary from one layer to the other, and the apparatus can be axially "deformed" in different manners, depending on such amounts. As is better explained hereafter, the connection between enclosures 6 provides a modular system, whose length may be adapted to different needs, by simply assembling an appropriate number of enclosures 6 and springs 7.

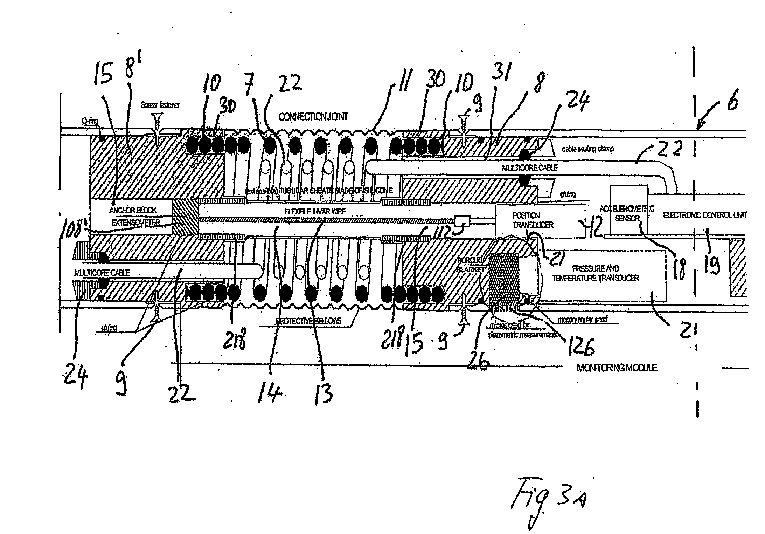

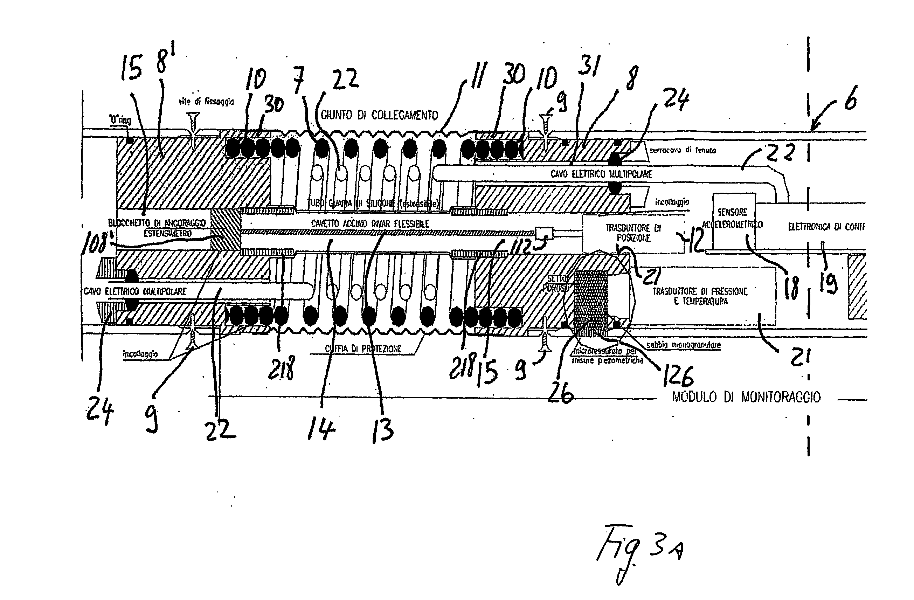

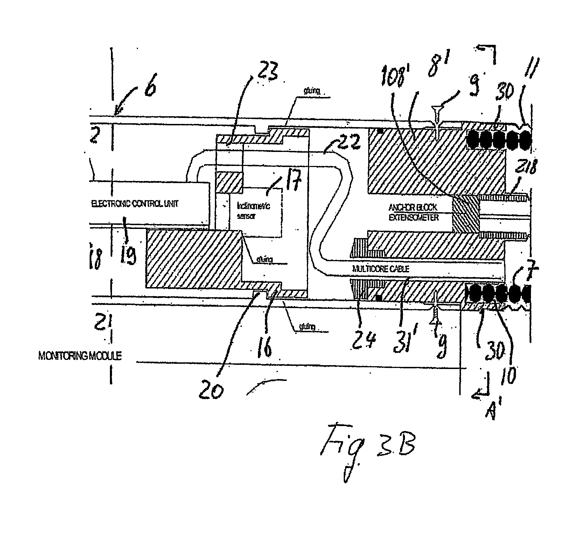

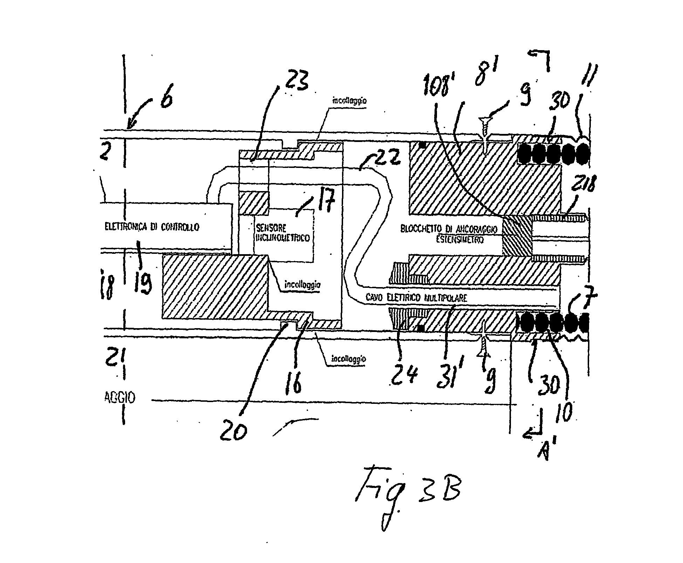

Referring now to Fig. 3, tubular enclosures 6 may have varying diameters and lengths, depending on the sensor/s to be housed therein, on the diameter and depth of the hole 1 and on the purposes of the geognostic survey. These enclosures may be made either of metal (such as stainless steel, aluminum, light metals) or plastic and synthetic materials (PVC, nylon, ABS, reinforced plastics, polycarbonates, or the like). Each enclosure 6 has at both ends a member 8, 8' for sealably closing the enclosure 6 and connecting it to the spring 7. The members 8, 8' are introduced in the enclosure from opposite ends, by forming a male/female coupling, and are fastened by peripheral locking screws 9. A rubber O-ring may be further provided. Each closing member 8, 8' has an annular groove 10, having a given axial extension, for accommodating one end of the spring 7, which extends from a member 8 to a member 8' immediately adjacent thereto and forms the connection between two adjacent enclosures 6. The helical springs 7 are preferably made of steel, and their elastic properties are suitable to the characteristics of each enclosure 6 and of the whole modular monitoring apparatus. Each spring is externally protected by a bellows 11, preferably made of rubber, whose opposite ends lie over the outer surface of the member 8, 8' on a peripheral flaring portion 30. This ensures the outward tightness, particularly liquid-tightness, of the whole apparatus, which is hermetically sealed all along its extension, except one or more very small portions, as described in greater detail below.

Each tubular enclosure 6 contains a position transducer 12, which may be kept in its axially central position on the closing member 8, by gluing or the like. This transducer 12 may be, for example, a permanent magnet which is capable of sliding in both directions, and which cooperates with an induction sensor for determining magnet displacement by magnetic induction. The magnet is subjected to the action of an elastic means which stably keeps it in the starting end-of-stroke position and restores such position upon return or cessation of the displacement stress. By way of example, the miniature LVDT straight position transducer, sold by RS, may be used. The purpose of this transducer 12 is to measure the amount of displacement of two adjacent enclosures 6 toward/away from each other. The mechanical connection for carrying the sensor of the position transducer 12 is obtained by a flexible wire 13, which may be made, for instance, of Invar or Kevlar, and is contained in an elastic sheath made of silicone, rubber or the like. The sheath further ensures the tightness of the system even when the bellows 11 is torn. The wire 13 is connected between the fork 112 of the transducer 12 and the axially central portion of the immediately adjacent closing member 8', by a coupling block 108', which is fastened in an axially central hole 15 of said member 8' and is held in position by inner radial projections 218, like the transducer 12. The closing member 8 also has a central axial hole 15 for the passage of the wire 13, which wire extends axially inside the helical spring 7. By gluing the transducer 12, tightness is ensured at the hole 15.

A holder 16 is provided in an intermediate portion of the enclosure 6, for supporting additional sensors 17, 18 and an electronic control card 19. The holder 16 has a circular section, whose diameter substantially corresponds to the inside diameter of the tubular enclosure 6, may be introduced in the enclosure 6 from the end that carries the member 8', to abut against an annular projection 20 provided on the inner surface of the enclosure 6. The holder 16 may be also fastened by using glue. It has a seat for fastening a tilt (inclinometer) sensor 17 and a hole 23 for the passage of a multicore cable 22, to be described in greater detail below. Regarding the tilt sensor 17, the biaxial electrolytic tilt sensor Spectron SP. An acceleration (accelerometer) sensor 18 is further fastened on the holder 16, to measure accelerometric variations induced by displacements, which sensor may be, for instance, the ADXL sensor sold by Analog Devices.

As mentioned above, the holder 16 further carries an electronic card 19, which is connected to all the sensors 12, 17, 18, 21 and has the function to collect and possibly locally process the data provided by the sensors 12, 17, 18, 21 in the enclosure 6. Each electronic card 19 further collects data from the electronic cards 19 in the previous tubular enclosures 6, down toward the substrate 4, and to transmit such data to the card 19 in immediately adjacent enclosures 6, to allow all detections to reach a local control unit 25 shared by the whole apparatus. Each electronic card 19 is also capable of receiving control and/or monitoring signals from the local unit 25 regarding one or more sensors 12, 17, 18, 21. Information is transferred among the various electronic cards 19 by a special multicore cable 22, which connects all the cards 19 in series, thereby forming a direct communication line. This cable 22 projects out of the two opposite ends of the enclosure 6 through two holes 31, 31' that are formed in each of the two end members 8, 8' respectively. At the inner end of each of the two holes 31, 31', a cable fastener 24, 24' is provided to ensure tightness even in case of accidental break of the protective bellows 11. At the connection between two adjacent enclosures 6, i.e. at the springs 7, the cable 22 is spiraled with a smaller radius than the springs, to accommodate the mutual displacements of the two enclosures 6, and to fit the motion of springs 7, with no risk of pinching. Therefore, the information from all electronic cards 19 reaches the control unit 25 or local unit, which may be housed in the first enclosure 6, i.e. the one in the proximity of the surface of the soil 3, or in a separate position, outside the borehole 1. Local electronic cards 19 have both functions of monitoring the sensors 12, 17, 18, 21 of each enclosure 6 and of acting as a connecting unit for the data and/or control transmission line which extends all along the apparatus, and passes through all the units formed by the enclosures 6. This feature, when combined with the construction features of the enclosures and the joints provides high modularity to the apparatus. In fact, the addition or removal of a unit - formed by an enclosure with the associated sensors and the electronic data transmission and control card - from an apparatus that contains a given number of these units is very simple, both mechanically and as regards the extension of the data transmit/receive line all along the apparatus, i.e. the electronic control and communication card of the new unit simply needs to be connected with the one of the terminal unit of the apparatus. The card may simply form the physical housing for a connector designed for extension of the data transmission line without having, as mentioned above, a section for communication control and management. This essentially depends on the selection of a transmission protocol from the group of currently available protocols.

The enclosure 6 further contains one or more pressure and/or temperature and/or moisture and/or piezometric level sensors. Suitable temperature sensors may be, for instance, those from the LM series by National Semiconductor, or from the TMP series by Analog Devices, whereas suitable piezoresistive pressure sensors may be for instance those sold by RS. The sensors for measuring this kind of parameters need to communicate directly with the outside. To this end, the sensitive portion of these one or more sensors 21 communicates with an external side recess of the enclosure 6, while being tightly isolated from the inside of the enclosure 6. The recess 26 contains a porous material, such as monogranular sand, and has a microslotted aperture 126 for communication with the outside.

The enclosure 6 may further contain other types of sensors, for detecting additional parameters. For example, a spontaneous potential detector may be provided, which may be a voltmeter, or the like, and/or a seismic detector.

The assembly of all the above elements forms the apparatus of the invention, which allows differential detection of the main physical and mechanical parameters of the relevant soil or structures.

The tubular enclosure 6' at the internal end of the apparatus, i.e. level with the stable substrate 4, may have an end closing member 8', acting as a sealing plug.

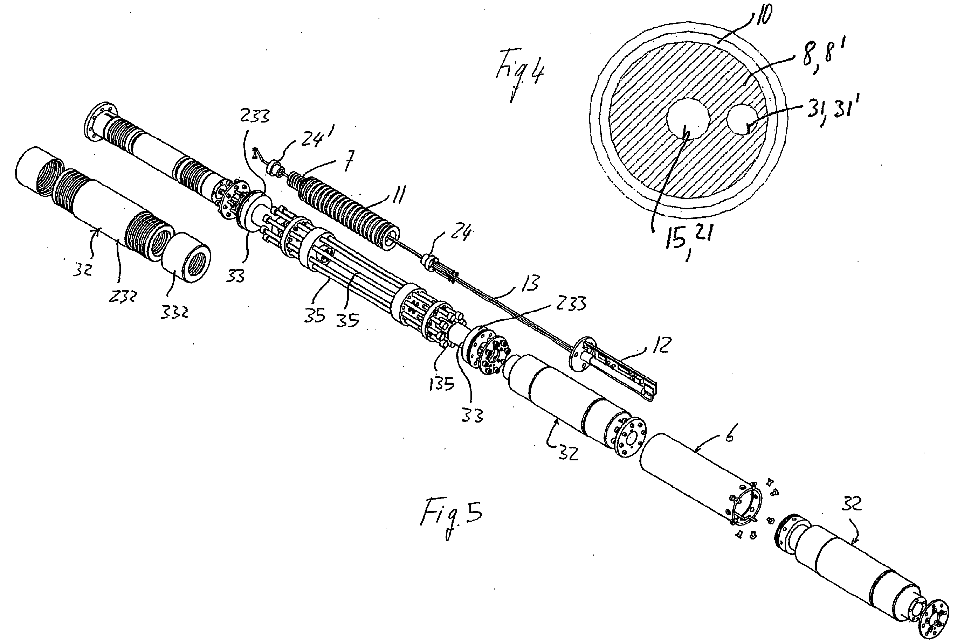

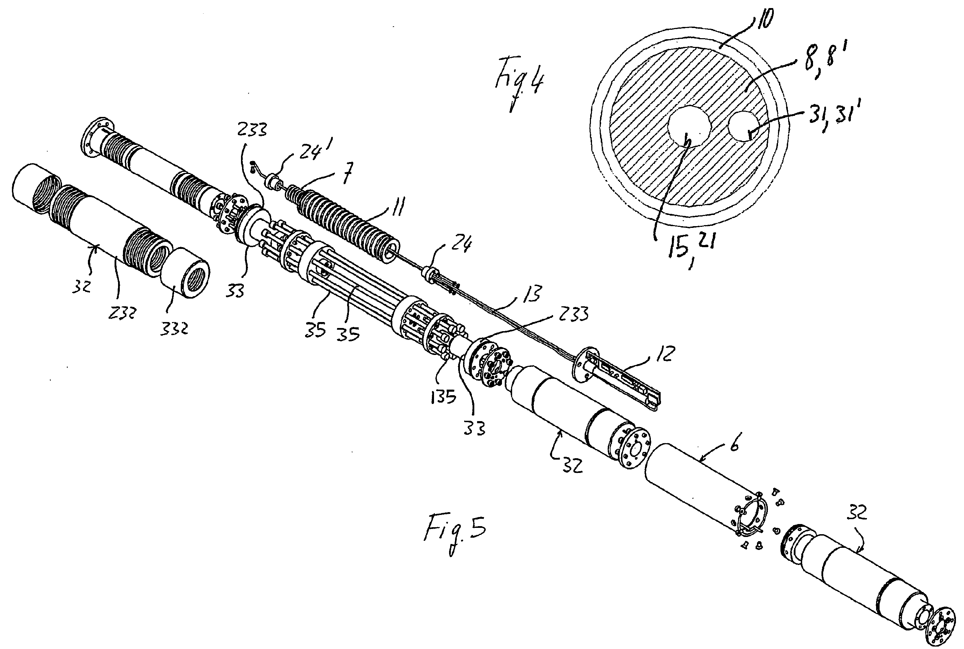

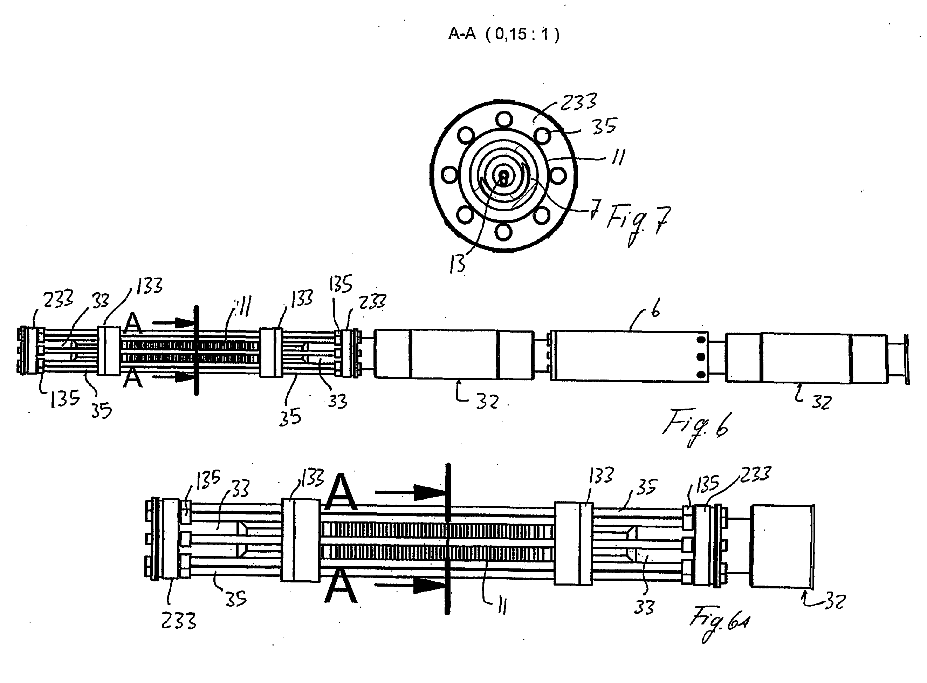

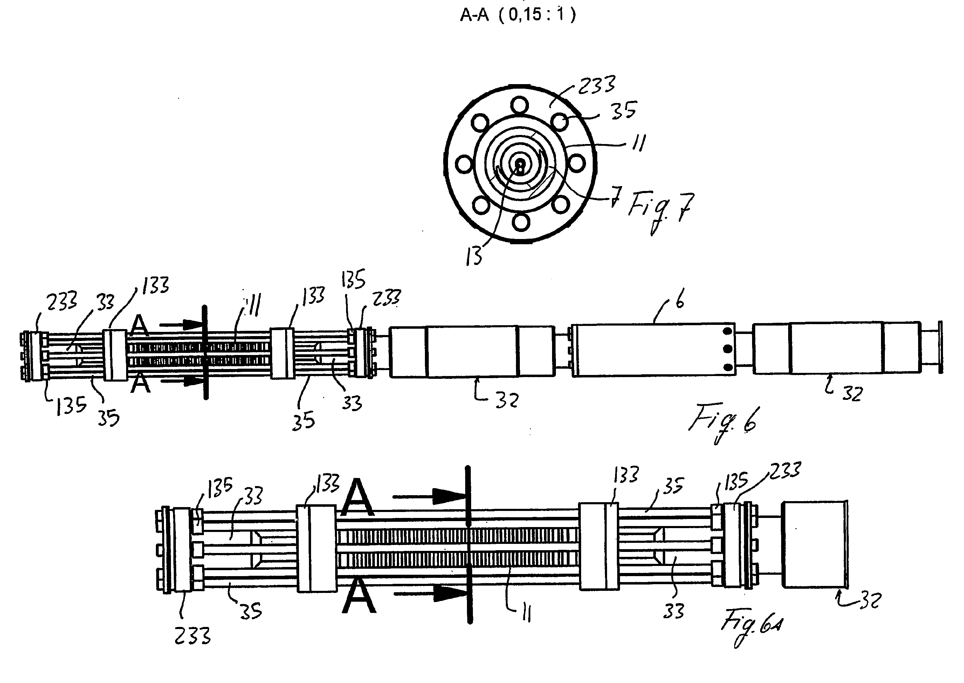

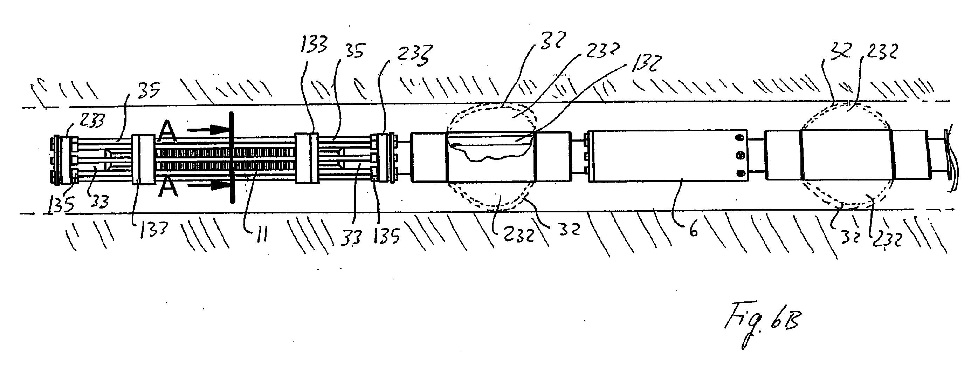

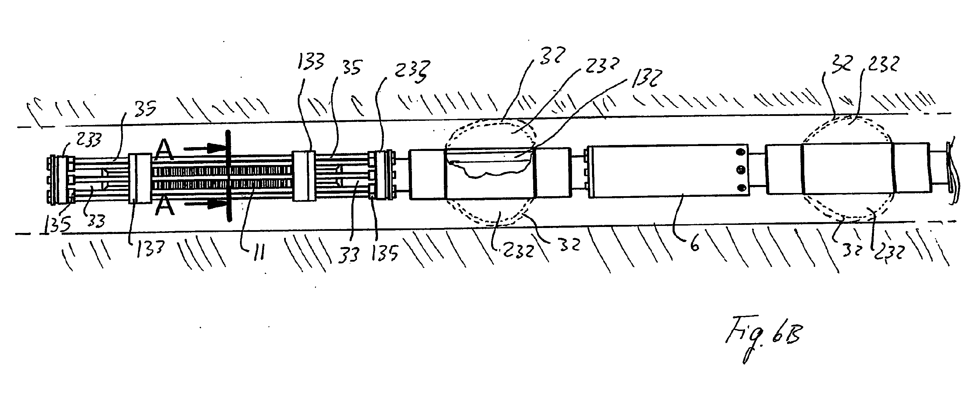

Referring now to Figs. 5 to 9, a second embodiment of the inventive apparatus is shown which, while providing essentially the same general functions as the first embodiment, further suggests some embodiments aimed at improving the behavior of the apparatus in certain situations. This embodiment also provides a succession of enclosures 6 and helical springs 7, whose respective construction features are essentially as described above regarding the first embodiment except that an anchor section is interposed between each opposite end of each enclosure 6 and the end of the immediately adjacent spring 7, for removably securing the device to the soil, rock or structure to be monitored. Therefore, an anchor section 32 is respectively provided immediately upstream and immediately downstream from each enclosure 6, which sections keep the enclosure 6 securely anchored to their respective portion of wall of the hole 1, whereas the portions of the apparatus which are level with the springs 7 are totally free to deform to accommodate any displacements of the soil 3. Nevertheless, while the design in the figures represents a preferred arrangement, described herein by way of example, a different arrangement and/or number of anchor sections 32 may be provided in each apparatus. Each section 32 has a substantially cylindrical shape, having essentially the same outside diameter and axial extension as the enclosures 6. However, sections 6 with different axial extensions or differentiated axial extensions within the same apparatus, may be provided, to adapt the behavior of the latter to the special stratigraphic composition of the soil. At one end, the section 32 is directly fastened to an end of the immediately adjacent enclosure 6 whereas a rigid terminal 33 for connection to the spring 7 is interposed between the opposite end and the spring 7, to accomplish another important function to be described in detail below. The anchor section 32 provides outward hermetic tightness, at least to liquids, and the connections with the enclosures 6 and the springs 7 are themselves designed in such a manner as to ensure an overall infiltration-tightness of the apparatus. Each anchor section 32 has an inner cylindrical wall 132 made of a rigid material, and an outer cylindrical wall 232 made of an extensible and expansible material, such as rubber or the like. The two walls 132, 232 are arranged coaxially and define a space therebetween wherein a pressurized fluid is fed, to cause outward radial expansion of the outer wall 232, as shown in Fig. 6C, which adheres against the inner surface of the hole 1, thereby locking the apparatus in position. One conduit 34 is provided for feeding pressurized fluid to the anchor sections 32, which conduit continuously extends within the apparatus and is connected at its outside end to a pressurized liquid source (not shown). In each anchor section 32, this conduit 34 is formed by perforating the thickness of the inner tubular inexpansible wall 232 parallel to the axis of the section 32, and has a few apertures 134 in communication with the space between the two walls 132, 232 for feeding fluid therein. The tubular shape of the anchor section 32 advantageously allows the axial passage of the wire or rod 13 for actuating the position transducer 12.

A further important characteristic of this second embodiment consists in that means for stopping the axial extension/compression stroke of the springs 7 are provided, which are a plurality of inextensible wires 35, particularly made of harmonic steel, which extend outside each spring protecting bellows 11, parallel thereto. Each of the opposite ends of each wire 35 has a widened head 135, which is slidably housed in a chamber formed in the rigid terminal 33 for connection of the spring 7 to the anchor member 32. The area of the expandable packer is formed by a chamber defined by an inner tubular wall 132 and an outer tubular wall 232. This chamber is provided in the form of a sleeve, and in the exploded view of Fig. 5 it is shown beside the wall 132, while the latter is fitted on the apparatus. The two tubular walls have end threads or corrugations which form tight joints for mutual clamping by using terminal ring nuts 332, which may be tightened or press-fitted on the tubular extensible wall 232, at these threaded or corrugated ends, that are complementary to each other. Other packer constructions may be obviously provided.

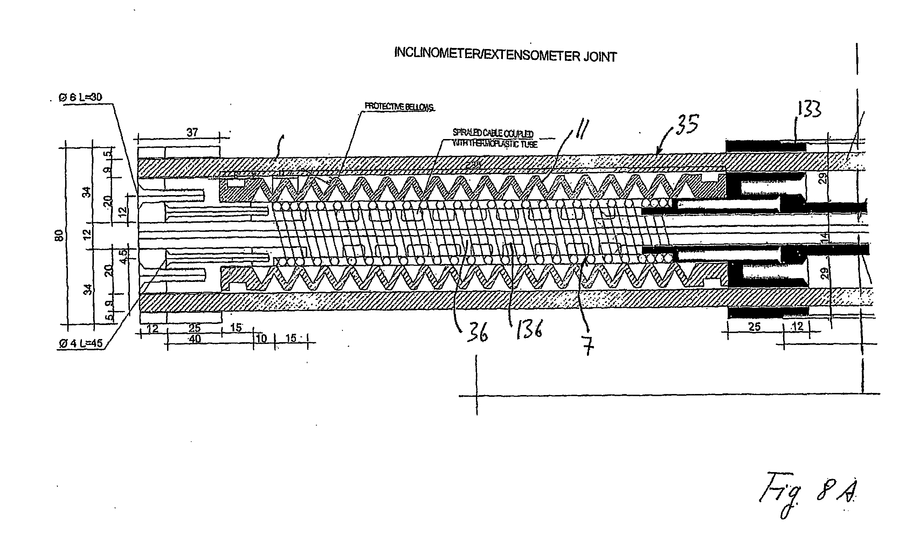

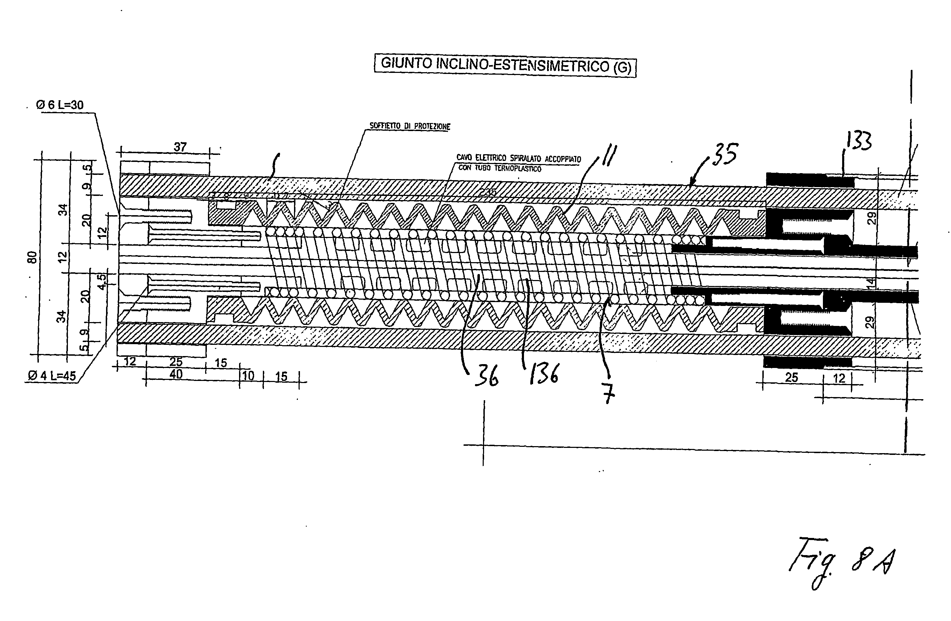

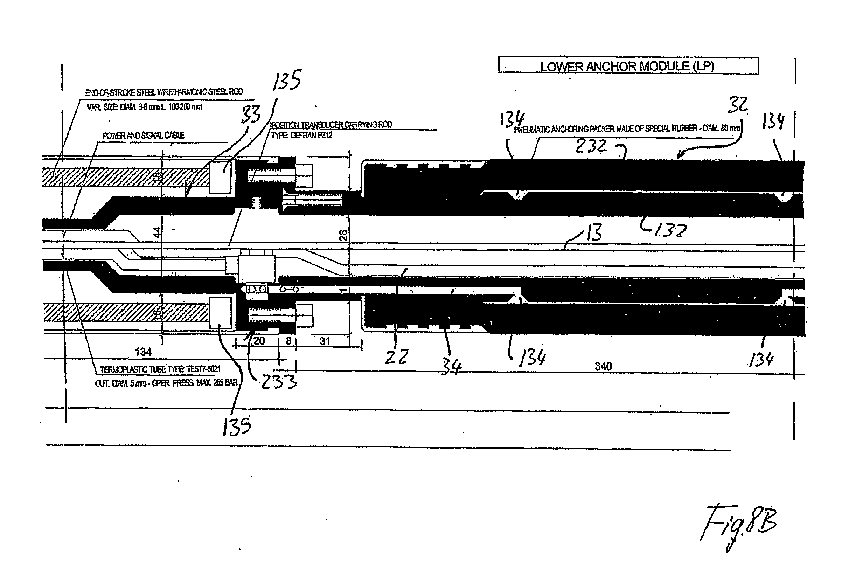

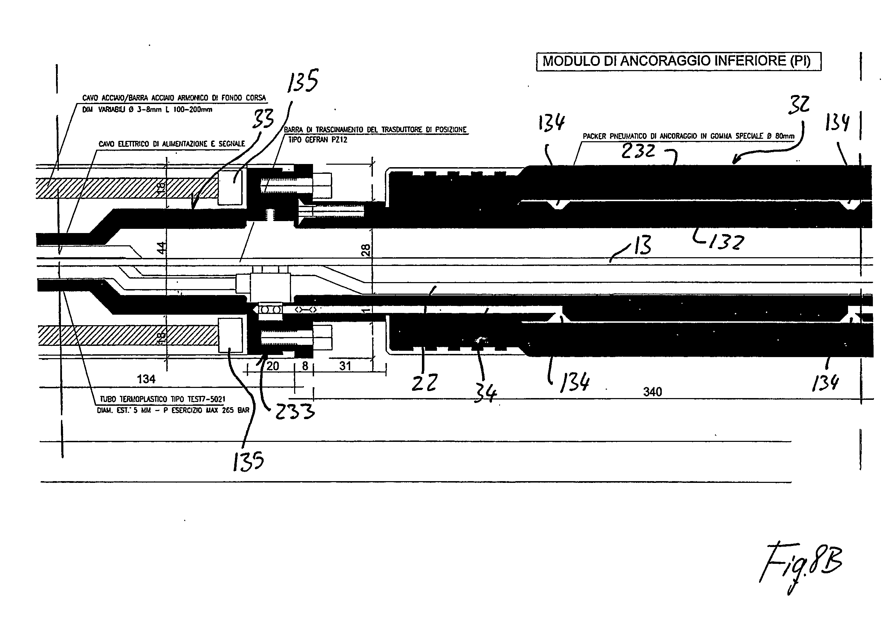

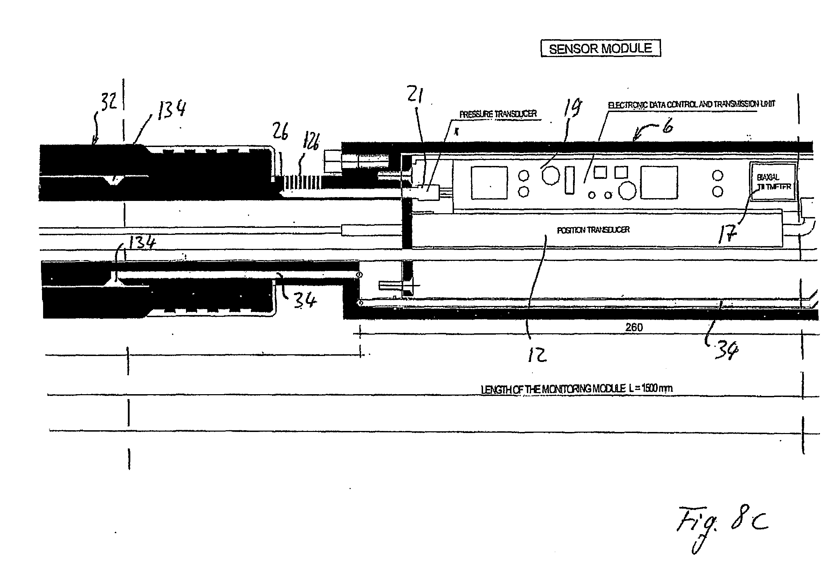

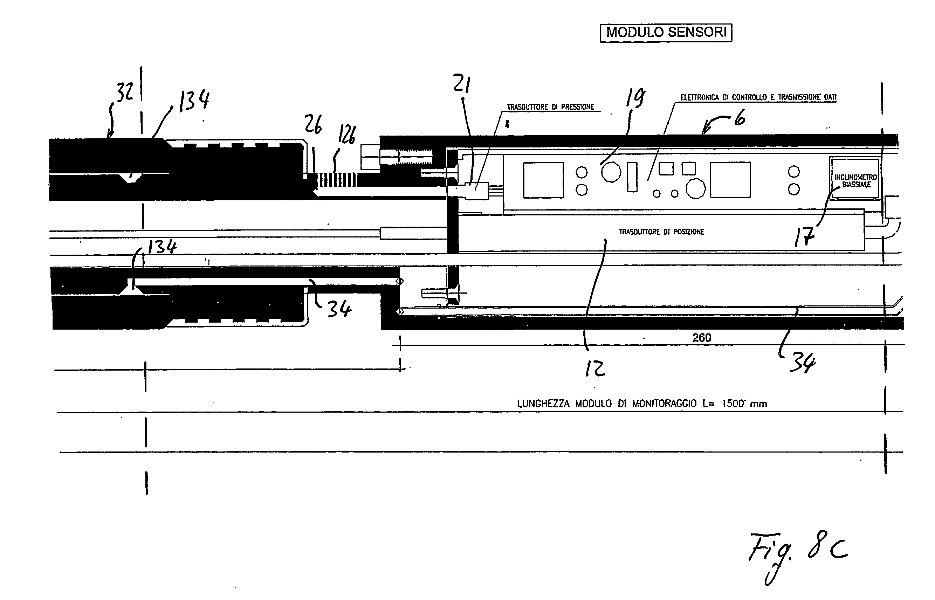

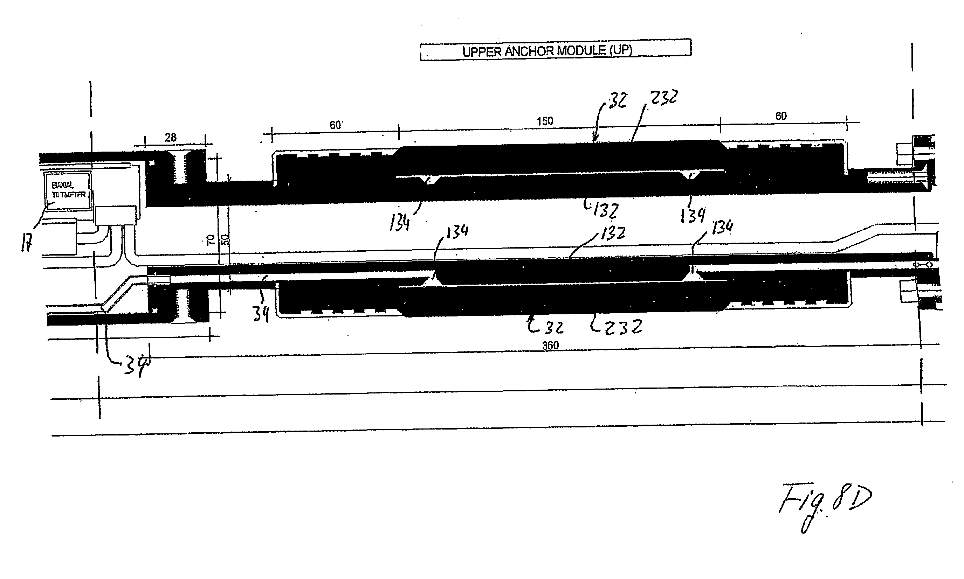

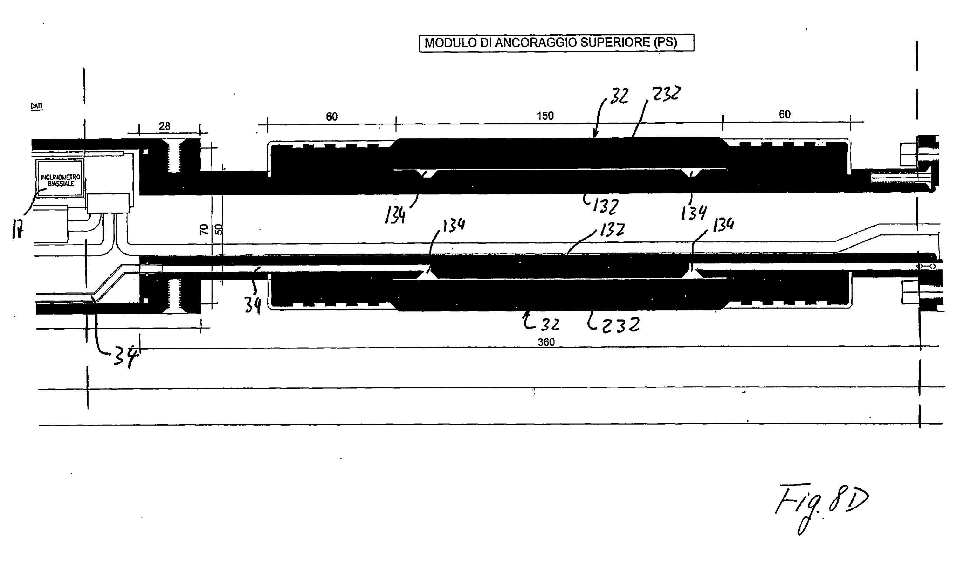

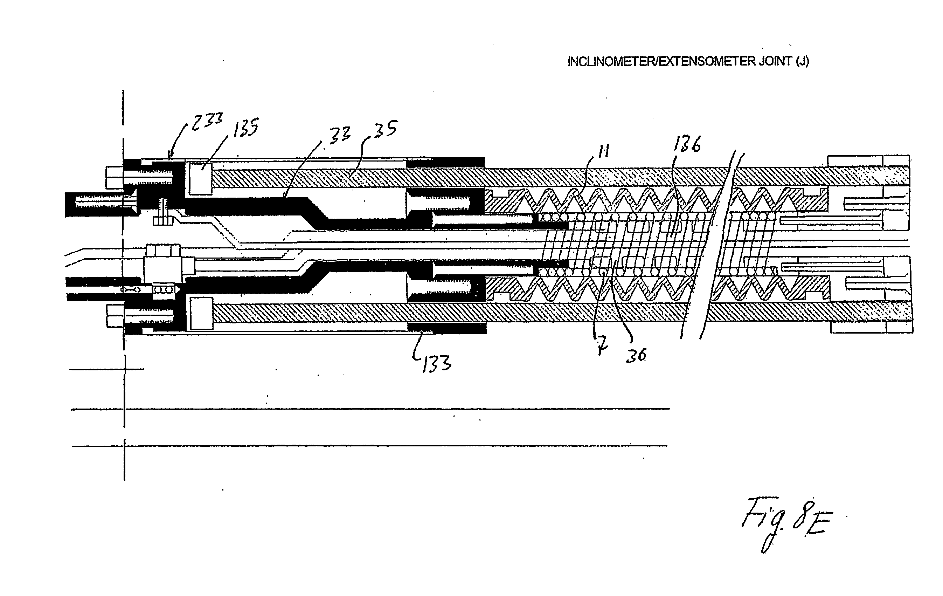

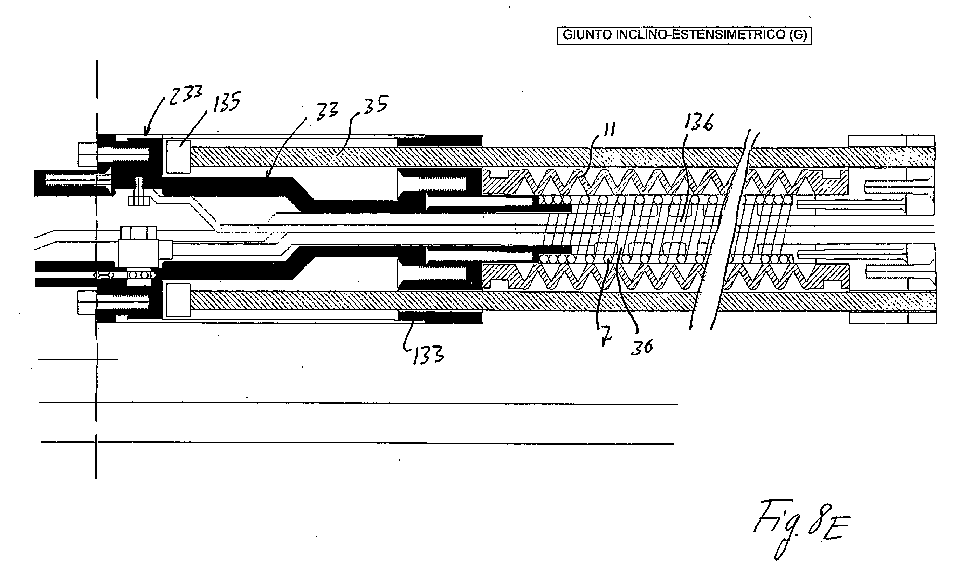

As shown in Figs. 8A to 8E, the terminal 33 progressively tapers toward the spring 7 whereto it is fastened, and the diameter difference between the terminal and the inspection hole 1 forms the chamber wherein the widened heads 135 slide. This chamber has two opposite flanges 133, 233, which form two opposite end-of-stroke abutments, for the extension and the compression of the spring 7 respectively. The use of steel wires 35 is particularly advantageous due to the inextensibility of this material, which maintains a sufficient flexibility to accommodate any side deformation of the spring 7. If the spring extends abnormally, the widened heads 135 of the wires 35 abut against the wall 133 and prevent any further extension of the spring, which might cause the permanent deformation or rupture thereof.

Like in the first embodiment, a continuous multicore cable 22 is provided for series connection of the various electronic cards 19 in the enclosures 6 that contain the sensors 12, 17, 18, 21. An element 36 is provided at the springs 7, to prevent any interference between the cable 22 and the feeding conduit 34 and between the latter and the spring 7, which element 36 extends axially from one end to the other of the spring 7, and is made of an elastically deformable material, to be axially compressible/extensible and deformable. This element 36 has a helical groove 135 on its outer surface for housing the feeding conduit 34 and the multicore transmission cable 22, to form a spiral winding core for both. Obviously, two separate spiraled grooves may be provided, for separately housing the conduit 34 and the cable 22.

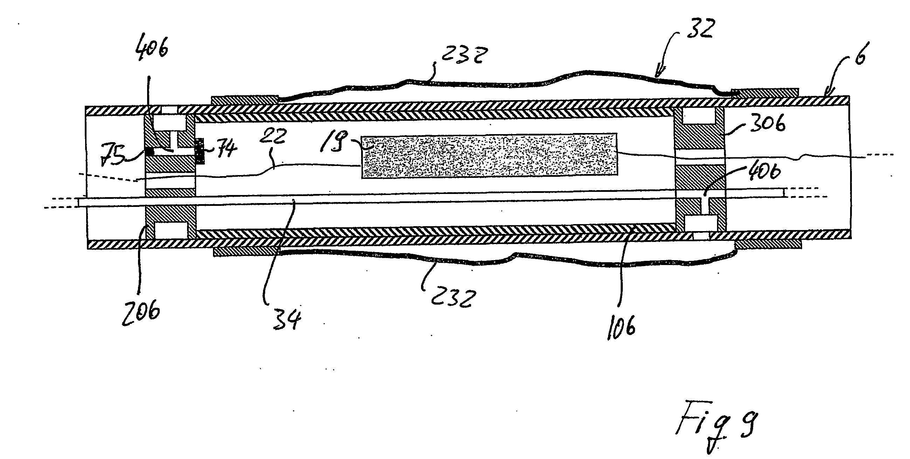

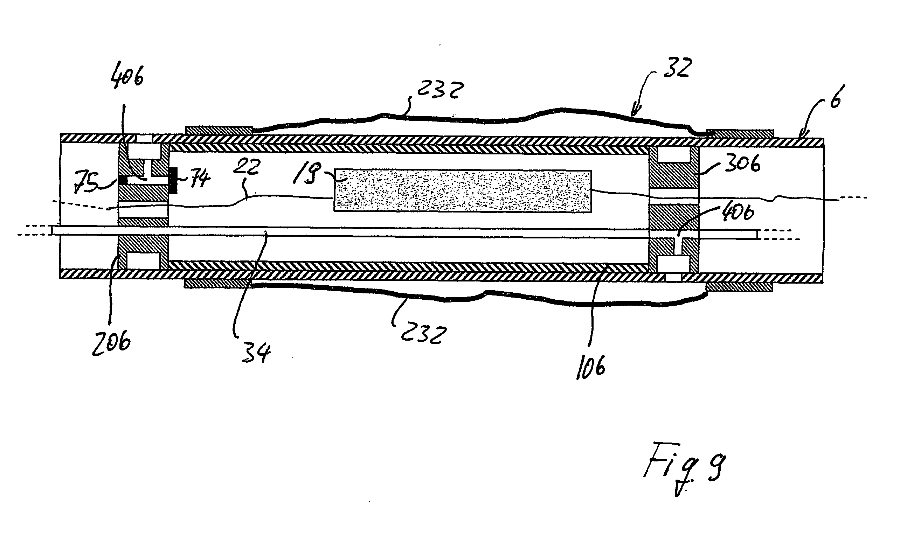

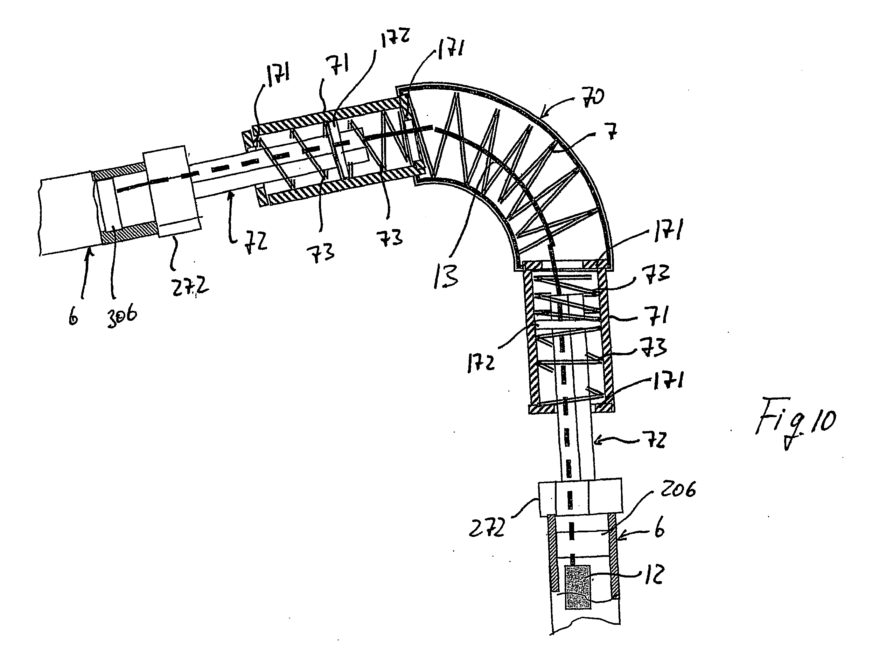

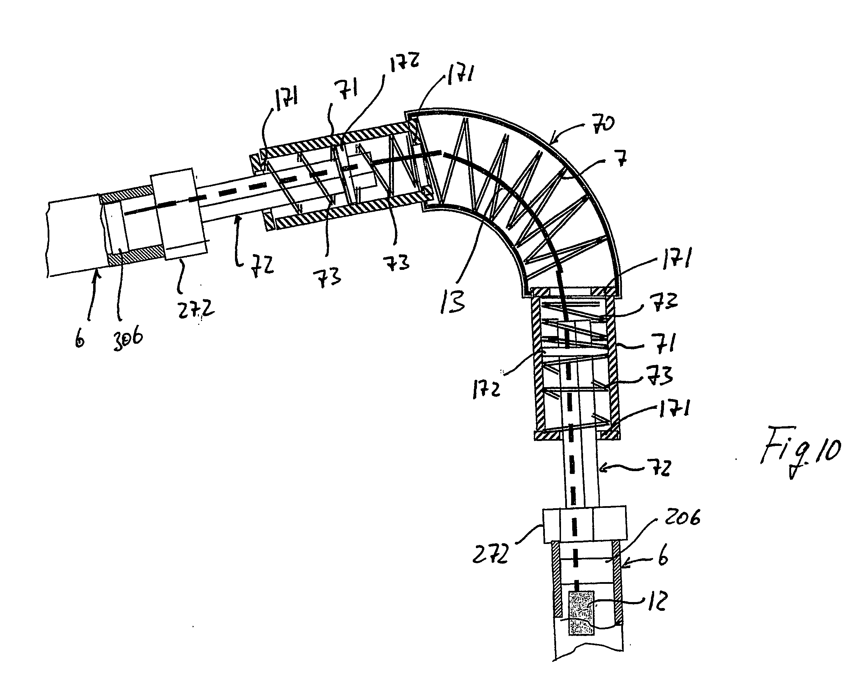

Figs. 9 and 10 show a third variant embodiment, which simplifies the above construction, by avoiding the presence of separate anchor sections 32 and a different type of deformable elastic joint. In this third embodiment, each enclosure 6 is formed by a tubular metal element, in the form of a sleeve or the like, which is externally fitted with anchor means 32, i.e. a tubular chamber surrounding the tubular metal element of the enclosure 6 and extending along a certain extension thereof. The anchor element 32 is made of an extensible material and the chamber thereof communicates with a conduit for feeding an expanding fluid, like water or air, which is pressure-fed through this conduit. Another rigid coaxial sleeve 106 is provided in the rigid tubular element 6, which sleeve forms at its ends an abutment step for two end closing members 206, 306, each being sealably coupled with the inner surface of the tubular element of the enclosure by means of an O-ring. The end closing members 206, 306 have at least one T-shaped conduit, with a radial stem opening on the peripheral outer surface of each closing member, preferably in a chamber that is formed by a groove or a peripheral recess, and with an axial stem opening on both ends of the closing member. The T-shaped conduit 406 is formed in the thickness of the closing members. In one case, and particularly for the closing member 306, the T-shaped conduit puts the expansion chamber of the anchor element 32 in communication with a tube for feeding an expansion fluid, such as pressurized water or air, which tube extends all along the enclosure until it reaches the opposite closing member 206, which has a through hole for the tube. Similarly, the two closing members 206, 306 have a through hole for the cables 22 connecting the various electronic measuring units in the enclosure 6 and possibly for the extensometer sensor pulling wire 13. The opposite closing member 206 has a T-shaped conduit that is closed on one of the two end sides and puts the environment outside the enclosure 6 in communication with a sealed space, which is either formed as a part of the chamber defined by the enclosure 6 or in the thickness of said closing member 206, and which houses a piezometric sensor for detection of water in the soil. In this case, the opening of the axial stem of the T-shaped conduit, on one side of the closing member 206 is sealed by a plug 75, whereas the opening turned toward the inside of the enclosure 6 is sealed by the piezometric element 76, which is connected to the electronic units in the enclosure 6 by suitable cables. This operating condition of the two closing members 206, 306 is achieved by providing that the closing member 206 has such a position, relative to the axial extension of the external enclosure 6 and anchor element 32 that said closing member 206 is in a portion of the enclosure 6 which is not covered by the anchor element 32. Advantageously, the closing members 206, 306 are provided in the form of pistons, with o-ring seals being provided between their outer peripheral surface and the inner surface of the enclosure 6 and/or of the tubular element 106 inside the enclosure 6, which forms the axial abutment steps for these two closing members 206, 306. As shown in the previous description, the closing members 206 and 306 are formed from a single construction part, which is mounted in the proper axial position for its function and is connected to the expansion fluid feeding tubes 34 for the anchor elements 32 and to the piezometric sensor respectively. Obviously, the two closing members 206, 306 are locked in an axial abutment position against their respective end step, which is formed by the sleeve 106 in the tubular enclosure 6, by fastener means, which one or more radial screws or in any other manner.

The two closing members 206, 306 further have a through hole for an extensometer sensor control wire. In this case, the wire is fastened to the closing member 206 or 306 of the enclosure 6 of an adjacent rigid module of the apparatus, extends through the elastic joint for connecting the latter to the previous rigid module, and passes into the enclosure 6 of this previous rigid module through the corresponding closing member 206 or 306 at the end of the enclosure 6 of this module, which faces toward the joint 1, in which enclosure the extensometer unit controlling cable is connected to the sensors of the extensometer unit.

In this variant, the elastic joints 7 between the various enclosure sections 6 are also constructed in a different manner, as shown in Fig. 10.

Particularly, each joint comprises a flexible sleeve 70, containing a spring 7. Two terminals 71 are fastened to the two ends of the flexible joint 70 and of the spring 7 respectively. The two terminals 71 have a tubular shape and each contain an axial slider 72, which has a radially widened portion 172, movable between two opposite radial abutments 171, which particularly have an annular shape, and are the ends of the terminals 71 in this particular embodiment. These end sides have a non-round central hole, for the passage of the accordingly non-round shaft of the slider 72, respectively housed in the terminal 71. The radially widened portion 172 of the slider allows the latter to slide axially along a stroke that essentially corresponds to the distance between the two radial abutments 171, whereas an elastic element, such as a helical spring 73, is provided between each side of the radial widened portion 172 of each slider 72 and the facing inner radial abutment 171 of the corresponding tubular terminal 71. Therefore, each slider is mounted with its radially widened portion 172 interposed between two helical springs. Outside the terminals 71, each slider 72 carries a coupling head 272, having a ring nut to be tightened to the corresponding measuring device. In order to ensure continuous electrical connection to the electronic measuring units and continuous feeding of expanding air or water to anchor elements 32, as well as the passage of the extensometer sensor control wire, connected on one side to the closing member 206, 306 of a tubular element 6 connected to an end of the joint 3, i.e. to a slider, and on the other side to the extensometer sensor in the tubular enclosure 6 of the module connected to the opposite slider 72 of the joint 3, the two sliders have an axial through hole for the passage of said wires, tubes and cables. Particularly, the tubes and cables and the extensometer sensor control wire coaxially pass through the helical spring 7. The cables and the tubes for feeding pressurized fluid to the anchor elements 32 advantageously have a helical design in this area which allows transverse bending and possibly elongation thereof.

Then, the coupling heads 27 of the sliders 72 are fastened to the facing ends of the tubular enclosures 6 of two adjacent successive rigid modules by using ring nuts.

According to a further improvement, the elastically extensible walls, and particularly the outer walls of said locking elements which come in contact with the soil may be reinforced with a braid or a mesh, particularly a metal braid or mesh. Such braid or mesh may be laid over the anchor section 32 or integrated in the material that forms at least the outer wall, designed to expand and be compressed against the soil. This embodiment of the extensible or flexible joints 3 allows to separate radial and axial axes thereof and to provide a dampening effect when tensile forces are applied on the apparatus, e.g. when the apparatus is removed from the borehole after extensive use. Furthermore, the non-round shafts of the sliders 72 allow to apply rotary forces to the apparatus to release it from the soil when it has to be extracted from the borehole.

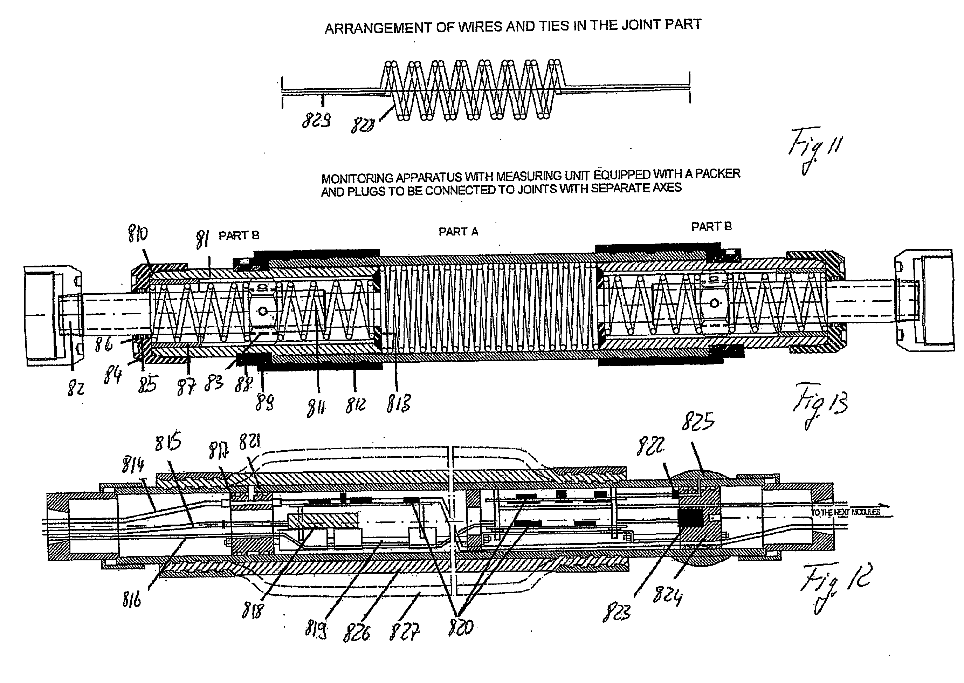

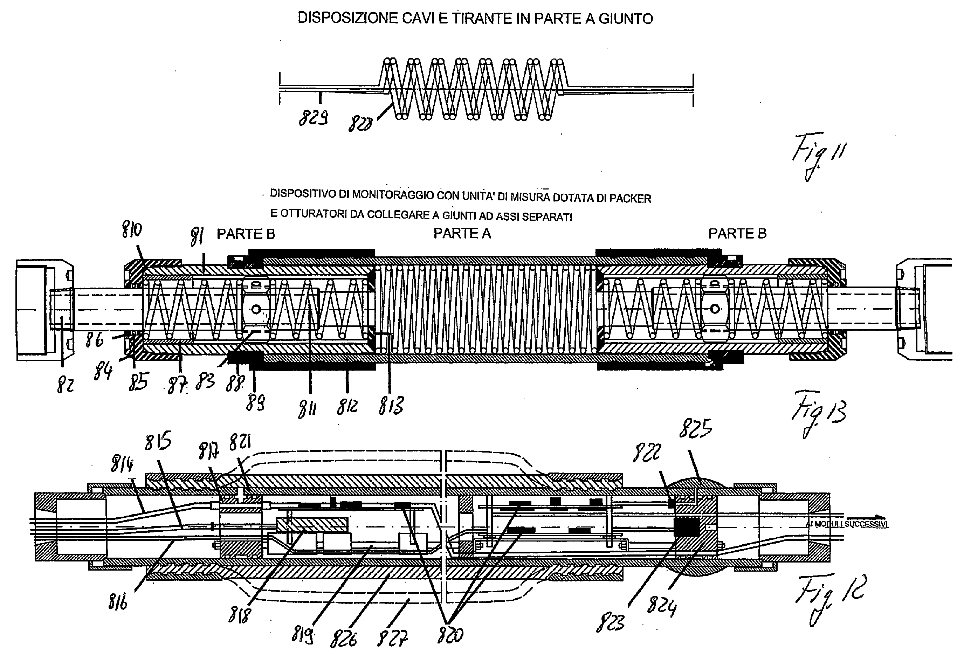

Fig. 11 shows the above design in greater detail. Figs. 12 and 13 show the monitoring apparatus with a measuring unit equipped with a packer, i.e. expandable anchor elements and plugs to be connected to joints with separate axes, as shown in Figure 13. Fig. 11 shows the arrangement of cables and ties in the joint of Fig. 13.

The figures clearly show the tubular body 81 having a hexagonal shape, the stainless steel shaft 82, the perforated hexagon 83, the ring nut 84 for connection to the rigid enclosures 6, the o-ring seals 85, and the Teflon rings 86 to promote sliding. A spacer 87 and two terminal fastening half-shells 88 are further provided. Numeral 89 designates a pressure ring for the tube and 810 designates further o-ring seals. The wire springs are designated by numeral 811 whereas 813 designates the bottom of the tubular sliding terminal which forms the end-of-stroke abutment. Numeral 812 designates the flexible tube covering the joint spring between the two terminals.

Numeral 814 designates the tube for feeding fluid to the packers and numeral 815 designates the tie associated to the extensometer sensor. Numeral 16 designates the power and signal transmission cable, whereas 817 designates the plug on the conduit for feeding expansion fluid to the packers. Numeral 181 designates the extensometer sensor. Numeral 819 designates the frame and supports for the electronic units, whereas 820 designates the electronic control units.

The piezometric and temperature sensors are designated by numeral 822, and the inclinometer sensor is designated by 823. Numeral 821 designates the straight inner tubular element of the tubular enclosure 6. Numeral 824 designates the pressure plug and 815 designates the porous filter at the opening of the tubular enclosure 6, allowing the passage of water to the piezometric sensor 822. Numeral 826 designates the wall of the packer, i.e. the expandable anchor device, in the idle condition, whereas 827 designates the wall in the expanded condition. Numeral 828 designates the power and signal cable and the tube for feeding the expansion fluid to the packers, whereas 829 designates the extensometer sensor driving tie.

Obviously, the invention is not limited to the embodiments described and illustrated herein, but may be greatly varied, especially as regards the number and arrangement of its elements, without departure from the guiding principle disclosed above and claimed below.

## Claims

1. An apparatus for monitoring geotechnical and structural parameters of soils (3), rocks and structures in general, comprising at least one enclosure (6), housing at least one sensor (12, 17, 18, 21) for at least one parameter, **characterized in that** it comprises at least two, preferably a plurality of rigid enclosures (6), each for housing at least one type of sensor (12, 17, 18, 21), which rigid enclosures (6) are connected by non-rigid connection members (7),
wherein
the enclosures (6) are arranged in succession one after the other along a predetermined line or a predetermined axis, at least one non-rigid connection member (7) being interposed between two immediately adjacent enclosures (6)
the connection members (7) allow a relative axial displacement of two adjacent enclosures (6) toward and/or away from each other and
a relative angular displacement of two adjacent enclosures (6),**characterised in that** ;
at least two adjacent enclosures (6) are connected by a tie (13) at least one of the ends of the tie (13) being dynamically connected to a sensor (12) which detects the relative axial position of the two enclosures (6) and which is housed in the said enclosures (6);
at least one inclination sensor (17) is provided in at least one of the enclosures (6).

2. An apparatus as claimed in claim 1, **characterized in that** each of the enclosures (6) and/or the non-rigid connecting members (7) are of the closed type, and impervious at least to external liquids.

3. An apparatus as claimed in claim 1 or 2, **characterized in that** the enclosures (6) are arranged alternate to the connection members (7).

4. An apparatus as claimed in one or more of the preceding claims, **characterized in that** the connection members (7) between the enclosures (6) are elastic, and restore the initial mutual enclosure (6) positioning condition, with reference to the at least one degree of freedom of motion or to all the degrees of freedom of motion of the enclosures (6) relative to each other.

5. An apparatus as claimed in one or more of the preceding claims, **characterized in that** the non-rigid connection members include elastic means (7) for restoring the initial idle or unstressed condition of the apparatus.

6. An apparatus as claimed in one or more of the preceding claims, **characterized in that** the enclosures (6) have a tubular, preferably cylindrical shape and particularly have equal diameters, and equal axial extensions.

7. An apparatus as claimed in one or more of the preceding claims, **characterized in that** the connection members (7) have an essentially tubular shape and extend along tubular, preferably cylindrical surfaces; particularly they may be formed by sections of steel braided rubber tubes, to withstand any angular deviation of the enclosures (6), caused by axial tensile and compression stresses, without imparting any rotation thereto.

8. An apparatus as claimed in claim 7, **characterized in that** the connection members (7) are helical springs (7) arranged coaxially to the tubular enclosures (6).

9. An apparatus as claimed in one or more of the preceding claims, **characterized in that** each helical spring (7) is fastened at its two opposite ends to the corresponding ends of two adjacent enclosures (6).

10. An apparatus as claimed in one or more of the preceding claims, **characterized in that** each helical spring (7) is outwardly hermetically covered by at least one tubular flexible sleeve, particularly bellows-shaped (11), preferably made of plastics, rubber or the like and possibly reinforced with a steel braid.

11. An apparatus as claimed in one or more of the preceding claims, **characterized in that** at least two adjacent enclosures (6) are connected by a tie (13) having such a length as to maintain the helical spring (7) in an intermediate pressure loading condition.

12. An apparatus as claimed in one or more of the preceding claims, **characterized in that** said sensor (12) is a permanent magnet which is capable of sliding in both directions toward the tie (13), which magnet cooperates with an induction sensor for determining magnet displacement by magnetic induction.

13. An apparatus as claimed in one or more of the preceding claims, **characterized in that** said sensor (12) is equipped with elastic means for pulling the magnet toward compression of the immediately adjacent connecting spring (7).

14. An apparatus as claimed in one or more of the preceding claims, **characterized in that** the tie (13) is of such a length as to keep the magnet of the enclosure (6) relative axial position sensor (12) in an intermediate signaling position when the apparatus is in an idle or unstressed position.

15. An apparatus as claimed in one or more of the preceding claims, **characterized in that** said tie is a wire (13) or a rod, particularly sheathed (14), and extends substantially along the central axis of the apparatus.

16. An apparatus as claimed in one or more of the preceding claims, **characterized in that** the enclosures (6), the connection members (7) and the connecting ties (13) between the enclosures (6) form an assembly of members to transfer soil (3) displacement stresses to position, pressure, inclination and acceleration sensors (12, 17, 18, 21) or transducers or the like.

17. An apparatus as claimed in one or more of the preceding claims, **characterized in that**, at each of their opposite ends, the enclosures (6) have a closing member (8, 8') which may be inserted and fastened therein, and acts as a terminal allowing attachment and/or passage of connecting springs (7), sealing sleeves (11), ties (13) and one or more position sensors (12).

18. An apparatus as claimed in one or more of the preceding claims, **characterized in that** at each opposite end of each enclosure (6), a substantially tubular rigid terminal (33) is provided for coupling each end of the enclosure (6) to the corresponding ends of the two immediately adjacent springs (7).

19. An apparatus as claimed in one or more of the preceding claims, **characterized in that** at least some of the connection members (7) have axial extension stop means (35).

20. An apparatus as claimed in one or more of the preceding claims, **characterized in that** at least some of the connection members (7) have axial compression stop means (35).

21. An apparatus as claimed in one or more of the preceding claims, **characterized in that** said extension or compression stop means are one or more inextensible elongate members (35), each extending inside or outside each spring (7), parallel to the axis thereof, one of the opposite ends (135) of said inextensible member (35) having a widened shape and being slidably accommodated inside a chamber having two opposite extension and compression stop abutments (133, 233), said chamber being provided within the closing member (8, 8') at the end of an adjacent enclosure (6) or within the substantially tubular rigid coupling terminal (33), whereas the opposite end of the inextensible member (53) is secured at the closing member (8, 8') or at the rigid member (33) for coupling the spring (7) to the other enclosure (6).

22. An apparatus as claimed in one or more of the preceding claims, **characterized in that** each of the opposite ends (135) of each inextensible member (35) has a widened shape and is slideably accommodated inside a chamber having two opposite extension and compression stop abutments (133, 233), each of said chambers being provided within each opposite closing member (8, 8') at each end of each enclosure (6) or within the substantially tubular rigid coupling terminals (33).

23. An apparatus as claimed in one or more of the preceding claims, **characterized in that** each of said inextensible stop members is a wire (35) or a rod, preferably made of harmonic steel, said inextensible members (35) being arranged along the outer circumference of the spring (7).

24. An apparatus as claimed in one or more of the preceding claims, **characterized in that**, when the apparatus is in the idle or unstressed condition, the widened ends (135) of each inextensible member (35) are in a spring (7) compression stop abutment position.

25. An apparatus as claimed in one or more of the preceding claims, **characterized in that** means (32) are provided for removably securing at least a portion of the apparatus to the soil (1), rock or structure to be monitored.

26. An apparatus as claimed in one or more of the preceding claims, **characterized in that** said securing means are one or more anchor sections (32), which sections (32) are arranged in a predetermined order relative to the enclosures (6) and to the connecting springs (7).

27. An apparatus as claimed in one or more of the preceding claims, **characterized in that** said anchor sections (32) are of the closed type and impervious to at least external liquids.

28. An apparatus as claimed in one or more of the preceding claims, **characterized in that** the locking action is obtained by radial expansion of the anchor sections (32) within the hole (1) in which the apparatus is housed.

29. An apparatus as claimed in one or more of the preceding claims, **characterized in that** an anchor section (32) is respectively provided immediately upstream and immediately downstream from each enclosure (6), each of said sections (32) being interposed between an end of the enclosure (6) and the substantially tubular rigid terminal (33) for connection to the immediately adjacent spring (7).

30. An apparatus as claimed in one or more of the preceding claims, **characterized in that** each anchor section (32) has a substantially tubular cylindrical shape, with substantially the same outside diameter as the enclosures (6), and is formed by an inner cylindrical wall (132) and an outer cylindrical wall (232) which are arranged coaxially and define a space therebetween, at least one of the two walls (132, 232), preferably the outer wall (232), being made of an extensible or expansible material, such as rubber or the like.

31. An apparatus as claimed in one or more of the preceding claims, **characterized in that** the locking action is achieved by an outward radial expansion of the outer wall (232) of each anchor section (32), which is obtained by introducing a pressurized fluid, in the empty space between the two walls (132, 232) of the section (32).

32. An apparatus as claimed in one or more of the preceding claims, **characterized in that** at least one conduit (34), particularly a single continuous conduit (34) is provided for feeding pressurized fluid to the anchor sections (32), which conduit (34) is connected at its outside end to a pressurized fluid source, and extends within each anchor section (32), parallel to the axis thereof, and has one or more radial apertures (134), which communicate with the empty space between the two walls (132, 232).

33. An apparatus as claimed in one or more of the preceding claims, **characterized in that** the feeding conduit (34) is formed by perforating the thickness of the inner non expandable tubular wall (132) of each anchor section (32).

34. An apparatus as claimed in one or more of the preceding claims, **characterized in that** said feeding conduit (34) extends within each enclosure (6), parallel to the axis thereof, or is obtained by perforating the thickness of the wall of the enclosure (6).

35. An apparatus as claimed in one or more of the preceding claims, **characterized in that**, level with the helical connecting springs (7), said conduit (34) is formed by a portion of flexible tube, particularly spiraled with a smaller radius than the springs themselves, to be able to accommodate deformations of springs (7) with no possible interference therewith.

36. An apparatus as claimed in one or more of the preceding claims, **characterized in that** one or more of the enclosures (6) contain, particularly in an intermediate position of their axial extension, at least one member (16) for carrying one or more sensors (12, 17, 18, 21).

37. An apparatus as claimed in one or more of the preceding claims, **characterized in that** at least one seismic sensor (17) is provided in at least one of the enclosures (6).

38. An apparatus as claimed in one or more of the preceding claims, **characterized in that** at least one temperature sensor (21) is provided in at least one of the enclosures (6).

39. An apparatus as claimed in one or more of the preceding claims, **characterized in that** at least one pressure or piezometric level sensor (21) is provided in at least one of the enclosures (6).

40. An apparatus as claimed in one or more of the preceding claims, **characterized in that** at least one electric potential meter is provided in at least one of the enclosures (6).

41. An apparatus as claimed in one or more of the preceding claims, **characterized in that** an electronic card (19) is provided in at least one of the enclosures (6), for collecting and/or locally processing the data provided by the sensors (12, 17, 18, 21) of the corresponding enclosure (6).

42. An apparatus as claimed in one or more of the preceding claims, **characterized in that** it comprises a multicore cable (22) for transmitting the data collected by sensors (12, 17, 18, 21), which extends from the last enclosure (6) to the first enclosure (6) of the succession of enclosures (6), and is successively led outside through a liquid-tight passage (31, 31').

43. An apparatus as claimed in one or more of the preceding claims, **characterized in that**, level with the helical connecting springs (7), the multicore transmission cable (22) is spiraled with a smaller radius than the springs, to ensure a perfect fit thereof with no risk of pinching.

44. An apparatus as claimed in one or more of the preceding claims, **characterized in that** the spring (7) winding contains at least one element (36) which extends axially from one end to the other of the spring (7), which element (36) is made of an elastically deformable material, to be axially compressible/extensible and deformable, at least one helical groove (136) being provided on the outer surface of said element (36), for accommodating the pressurized fluid feeding conduit (34) and/or the multicore transmission cable (22).

45. An apparatus as claimed in one or more of the preceding claims, **characterized in that** the multicore transmission cable (22) is discontinuous and connects the cards (19) for collecting the data provided by the sensors (12, 17, 18, 21) associated to the enclosures (6), said data collection cards (19) having inputs and outputs for the transmission line, and a receive and transmit section based on multichannel or multitasking protocols or transmission buses.

46. An apparatus as claimed in one or more of the preceding claims, **characterized in that** the temperature sensor (21) and/or the pressure sensor may communicate with the outside of their respective enclosures (6), the sensitive portion of said sensors (21) being housed or communicating with an outer side recess (26) or undercut of the enclosure (6), while being hermetically isolated from the inside of the enclosure (6).

47. An apparatus as claimed in one or more of the preceding claims, **characterized in that** the peripheral wall of the enclosure (6) has an outer wall (126) with microslots at the recess (26) for communication between the sensors (21) and the outside.

48. An apparatus as claimed in one or more of the preceding claims, **characterized in that** the recess (26) used by temperature and/or pressure sensors (21) to communicate with the outside is filled with a porous material.

49. An apparatus as claimed in one or more of the preceding claims, **characterized in that** a local data collection control unit (25) is provided, which is connected to the transmission cable (22) and transmit controls to the data collection cards (19) and/or the sensors (12, 17, 18, 21).

50. An apparatus as claimed in one or more of the preceding claims, **characterized in that** the local unit further has means for radio transmission/reception of controls and data to/from a central supervision location.

51. An apparatus as claimed in one or more of the preceding claims, **characterized in that** the local unit (25) may be programmable relative to the functions to be accomplished, such as sensor actuating and/or reading times (12, 17, 18, 21), alarm thresholds or the like.

52. An apparatus as claimed in one or more of the preceding claims, **characterized in that** the local control unit (25) may be separate or integrated in a first enclosure (6).

53. An apparatus as claimed in one or more of the preceding claims, **characterized in that** the flexible joints (3) have ends that axially slide (72) between two extreme end-of-stroke positions (171), which sliding ends are subjected to elastic forces opposing the axial displacement, providing a dampening effect.

54. An apparatus as claimed in one or more of the preceding claims, **characterized in that** the expandable anchor elements (32) are placed level with the rigid tubular enclosures (6).

55. An apparatus as claimed in claim 54, **characterized in that** the anchor elements 32 have a tubular shape and lie over the outer surface of the rigid enclosures (6) along at least a portion of the axial extension thereof and/or over at least a portion of the circumferential extension of said outer surface of the rigid tubular enclosures (6).

56. An apparatus as claimed in claim 54 or 55, **characterized in that** the expandable anchor elements (32) are fed with pressurized fluid through the outer wall of the rigid tubular enclosure (6), by means of an angled conduit, having an axial stem and a radial stem, which is formed in an end closing member (306) of the tubular enclosure (6), and which conduit communicates, by its axial stem, with a pressurized fluid feeding tube and by its radial stem, with a through aperture formed in the outer surface of the rigid tubular element (6), which aperture coincides and is sealably connected with a feeding aperture on the adjacent contact surface of the anchor element (32) against the outer surface of the rigid tubular enclosure (6).

57. An apparatus as claimed in one or more of the preceding claims 54 to 56, **characterized in that** each joint (3) is composed of a flexible helical spring (7), which is housed in a flexible sleeve whose ends are formed by terminals (71) acting as sliding rollers for respective axial sliders (72), each having a non-round shaft which passes through a complementary non-round hole in said terminals (71) and whose end within the respective terminal (71) is radially widened (172) and abuts against two axially spaced radial inner stops (171), an elastic member, for instance a helical spring being provided between the radially widened portion (172) of the slider (72) and the radial inner stops (171) of the corresponding terminal (71).

58. An apparatus as claimed in one or more of the preceding claims 54 to 57 , **characterized in that** axial through holes are formed in the shafts of the axial sliders (72) of the joints (3).

59. An apparatus as claimed in one or more of the preceding claims 54 to 58, **characterized in that** the outside ends of the axial sliders (72) have means for mechanically fastening them to respective modules, i.e. rigid tubular enclosures (6).

## Patentansprüche

1. Vorrichtung zur Überwachung von geotechnischen und strukturellen Parametern von Böden (3), Gestein und Strukturen allgemein, umfassend wenigstens eine Einschließung (6), unterbringend wenigstens einen Sensor (12, 17, 18, 21) für wenigstens einen Parameter, **dadurch gekennzeichnet, dass** sie mindestens zwei, bevorzugt eine Mehrzahl von starren Einschließungen (6) enthält, jeweils zur Unterbringung mindestens eines Typs von Sensor (12, 17, 18, 21), wobei diese starren Einschließungen (6) durch unstarre Verbindungselemente (7) verbunden sind,
wobei die Einschließungen (6) in der Folge hintereinander entlang einer vorgegebenen Linie und/oder einer vorgegebenen Achse angeordnet sind, wobei mindestens ein unstarres Verbindungselement (7) zwischen zwei unmittelbaren aneinandergrenzenden Einschließungen (6) zwischengeschaltet ist,
die Verbindungselemente (7) eine relativ axiale Verlagerung zweier aneinandergrenzenden Einschließungen zueinander und/oder voneinander weg zulassen und
eine relativ winkelige Verlagerung zweier aneinandergrenzenden Einschließungen, **dadurch gekennzeichnet, dass**
mindestens zwei aneinandergrenzende Einschließungen (6) durch eine Bindung (13) verbunden sind, wobei wenigstens eines der Enden der Bindung (13) mit einem Sensor dynamisch verbunden ist, der die relativ Axialposition beider Einschließungen (6) erfasst und das in diesen Einschließungen (6) untergebracht ist;
zumindest ein Neigungssensor (17) in wenigstens einer der Einschließungen (6) vorhanden ist.

2. Vorrichtung nach Anspruch 1, **dadurch gekennzeichnet, dass** die Einschließungen (6) und/oder die unstarren Verbindungselemente (7) jeweils geschlossen gestaltet und wenigstens äußeren Flüssigkeiten standfest ist.

3. Vorrichtung nach Anspruch 1 oder 2, **dadurch gekennzeichnet, dass** die Einschließungen (6) alternativ zu den Verbindungselementen (7) angeordnet sind.

4. Vorrichtung nach einem oder mehreren vorhergehenden Ansprüchen, **dadurch gekennzeichnet, dass** die Verbindungselemente (7) zwischen den Einschließungen (6) elastisch sind und die anfängliche gegenseitige Positionierungsbedingung der Einschließungen (6) wiederherstellen, mit Bezugnahme auf den mindestens einen Bewegungsfreiheitsgrad oder auf alle Bewegungsfreiheitsgrade der Einschließungen (6) relativ zueinander.

5. Vorrichtung nach einem oder mehreren vorher- gehenden Ansprüchen, **dadurch gekennzeichnet, dass** zur Wiederherstellung des anfänglichen leerlaufenden oder unbelasteten Zustands der Vorrichtung die unstarren Verbindungselemente elastische Mittel (7) enthalten.

6. Vorrichtung nach einem oder mehreren vorher- gehenden Ansprüchen, **dadurch gekennzeichnet, dass** die Einschließungen (6) eine rohrförmige, vorzugsweise zylinderförmige Form und insbesondere gleichmäßige Durchmesser und gleichmäßige axiale Ausdehnungen aufweisen.

7. Vorrichtung nach einem oder mehreren vorhergehenden Ansprüchen, **dadurch gekennzeichnet, dass** die Verbindungselemente (7) eine im Wesentlichen Rohrform aufweisen und sich entlang von rohrförmigen, vorzugsweise zylinderförmigen Oberflächen erstrecken; sie können insbesondere aus Abschnitten von stahlgeflochtenen Gummischläuche bestehen, um jeglicher Winkelabweichung der Einschließungen (6) zu widerstehen, infolge der axialen Zug- und Druckbelastungen, ohne dieser jegliche Drehung zu vermitteln.

8. Vorrichtung nach Anspruch 7, **dadurch gekennzeichnet, dass** die Verbindungselemente (7) koaxial zu den rohrförmigen Einschließungen (6) angeordnet sind.

9. Vorrichtung nach einem oder mehreren vorhergehenden Ansprüchen, **dadurch gekennzeichnet, dass** jede Schraubenfeder (7) an ihren beiden entgegengesetzten Enden an den entsprechenden Enden zweier aneinandergrenzenden Einschließungen (6) befestigt sind.

10. Vorrichtung nach einem oder mehreren vorhergehenden Ansprüchen, **dadurch gekennzeichnet, dass** jede Schraubenfeder (7) von mindestens einer rohrförmigen flexiblen Hülse nach außen hin dicht abgedeckt ist, wobei diese Hülse insbesondere faltenbalgförmig (11) ist, vorzugsweise aus Kunststoff, Gummi oder dergleichen bestehen und gegebenenfalls mit einem Stahlgeflecht verstärkt ist.

11. Vorrichtung nach einem oder mehreren vorhergehenden Ansprüchen, **dadurch gekennzeichnet, dass** mindestens zwei aneinandergrenzende Einschließungen durch eine Bindung (13) angeschlossen sind, deren Länge derart ist, um die Schraubenfeder (7) in einem Zwischendruckbelastungszustand beizubehalten.

12. Vorrichtung nach einem oder mehreren vorhergehenden Ansprüchen, **dadurch gekennzeichnet, dass** dieser Sensor (12) ein Dauermagnet ist, der in beide Richtungen nach der Bindung (13) gleiten kann, wobei zur Ermittlung der magnetischen Verlagerung dieser Magnet mit einem Induktionssensor zusammenwirkt.

13. Vorrichtung nach einem oder mehreren vorhergehenden Ansprüchen, **dadurch gekennzeichnet, dass** dieser Sensor (12) mit elastischen Mitteln ausgestattet ist, um den Magnet zu ziehen und die unmittelbar aneinandergrenzende Verbindungsfeder (7) zu verdichten.

14. Vorrichtung nach einem oder mehreren vorhergehenden Ansprüchen, **dadurch gekennzeichnet, dass** die Bindung (13) eine derartige Länge aufweist, um den Magnet des Sensors (12) für die relative Axialposition der Einschließung (6) in einer Zwischenmeldeposition beizubehalten, wenn sich die Vorrichtung in einer leerlaufenden oder unbelasteten Position befindet.

15. Vorrichtung nach einem oder mehreren vorher- gehenden Ansprüchen, **dadurch gekennzeichnet, dass** diese Bindung ein Draht (13) oder eine Stange, insbesondere eine gehüllte Stange (14) ist und sich im Wesentlichen entlang der Mittelpunktachse der Vorrichtung erstreckt.

16. Vorrichtung nach einem oder mehreren vorhergehenden Ansprüchen, **dadurch gekennzeichnet, dass** die Einschließungen (6), die Verbindungselemente (7) und die Anschlussbindungen (13) zwischen den Einschließungen (6) einen Satz von Elementen bilden, um Verlagerungsbelastungen des Bodens (3) an Position-, Druck-, Neigungs- und Beschleunigungssensoren (12, 17, 18, 21) oder Messwandler oder dergleichen zu übertragen.

17. Vorrichtung nach einem oder mehreren vorhergehenden Ansprüchen, **dadurch gekennzeichnet, dass** an jedem ihrer entgegengesetzten Enden die Einschließungen (6) ein Verschlusselement (8, 8') haben, das in diesem einbringbar und befestigbar ist, und wenn eine Anschlussklemme wirkt, um die Befestigung und/oder den Durchgang von Verbindungsfedern (7), Abdichtungshülsen (11), Bindungen (13) und einem oder mehreren Positionssensoren (12) zu ermöglichen.

18. Vorrichtung nach einem oder mehreren vorhergehenden Ansprüchen, **dadurch gekennzeichnet, dass** an jedem entgegengesetzten Ende jeder Einschließung (6), eine wesentlich rohrförmige starre Anschlussklemme (33) zur Kupplung jedes Endes der Einschließung (6) mit den entsprechenden Enden der zwei unmittelbar aneinandergrenzenden Federn (7) angeordnet ist.

19. Vorrichtung nach einem oder mehreren vorhergehenden Ansprüchen, **dadurch gekennzeichnet, dass** zumindest einige der Verbindungselemente (7) Arretierungsmittel (35) für die Axialausdehnung haben.

20. Vorrichtung nach einem oder mehreren vorhergehenden Ansprüchen, **dadurch gekennzeichnet, dass** zumindest einige der Verbindungselemente (7) Arretierungsmittel (35) für die Axialverdichtung haben.

21. Vorrichtung nach einem oder mehreren vorhergehenden Ansprüchen, **dadurch gekennzeichnet, dass** diese Arretierungsmittel für die Ausdehnung oder Verdichtung ein oder mehrere undehnbare langgestreckte Elemente (35) sind, wobei jedes sich innerhalb oder außerhalb von jeder Feder (7) parallel zu deren Achse erstreckt, wobei eines der entgegengesetzten Enden (135) dieses undehnbaren Elements (35) eine aufgeweitete Form aufweist und innerhalb einer Kammer untergebracht ist, die zwei entgegengesetzten Arretierungswiderlager (133, 233) für die Ausdehnung und die Verdichtung enthält, wobei diese Kammer innerhalb des Verschlusselements (8, 8') am Ende einer aneinandergrenzenden Einschließung (6) oder innerhalb der im Wesentlichen rohrförmigen starren Kupplungsklemme (33) angeordnet ist, wobei das entgegengesetzte Ende des undehnbaren Elements (53) am Verschlusselemente (8, 8') oder am starren Elemente (33) zur Kupplung der Feder (7) mit der anderen Einschließung (6) gesichert ist.

22. Vorrichtung nach einem oder mehreren vorhergehenden Ansprüchen, **dadurch gekennzeichnet, dass** die entgegengesetzten Enden (135) jedes undehnbaren Elements (35) jeweils eine aufgeweitete Form aufweisen und innerhalb einer Kammer mit zwei entgegengesetzten Arretierungswiderlagern (133, 233) für die Ausdehnung und die Verdichtung gleitend untergebracht ist, wobei diese Kammer jeweils innerhalb jedes entgegengesetzten Verschlusselements (8, 8') an jedem Ende einer Einschließung (6) oder innerhalb der im wesentlichen rohrförmigen starren Kupplungsklemmen (33) angeordnet ist.

23. Vorrichtung nach einem oder mehreren vorhergehenden Ansprüchen, **dadurch gekennzeichnet, dass** jedes dieser undehnbaren Arretierungselemente ein Draht (35) oder eine Stange, vorzugsweise aus Federstahl, ist, wobei diese undehnbaren Elemente (35) entlang des Außenumfangs der Feder (7) angeordnet sind.

24. Vorrichtung nach einem oder mehreren vorhergehenden Ansprüchen, **dadurch gekennzeichnet, dass** die aufgeweiteten Enden (135) jedes undehnbaren Elements (35) in einer Widerlagerposition sind, in welcher die Arretierung der Verdichtung der Feder (7) erfolgt, wenn sich die Vorrichtung in einem leer- laufenden oder unbelasteten Zustand befindet.

25. Vorrichtung nach einem oder mehreren vorhergehenden Ansprüchen, **dadurch gekennzeichnet, dass** Mittel (32) zur entnehmbaren Sicherung wenigstens eines Teils der Vorrichtung vom zu überwachenden Boden (1), vom Gestein oder von der Struktur vorhanden sind.

26. Vorrichtung nach einem oder mehreren vorhergehenden Ansprüchen, **dadurch gekennzeichnet, dass** diese Sicherungsmittel ein oder mehrere Ankerabschnitte (32) sind, wobei diese Abschnitte (32) in einer vorgegebenen Ordnung im Verhältnis zu den Einschließungen (6) und zu den Verbindungsfedern (7) angeordnet sind.

27. Vorrichtung nach einem oder mehreren vorhergehenden Ansprüchen, **dadurch gekennzeichnet, dass** diese Ankerabschnitte (32) geschlossen gestaltet und standfest zu den wenigstens äußeren Flüssigkeiten standfest sind.

28. Vorrichtung nach einem oder mehreren vorhergehenden Ansprüchen, **dadurch gekennzeichnet, dass** die Verriegelung durch radiale Dehnung der Ankerabschnitte (32) innerhalb des Loches (1) erfolgt, in welchem die Vorrichtung untergebracht ist.

29. Vorrichtung nach einem oder mehreren vorhergehenden Ansprüchen, **dadurch gekennzeichnet, dass** ein Ankerabschnitt (32) jeweils unmittelbar aufwärts und unmittelbar abwärts von jeder Einschließung (6) vorhanden ist, wobei jeder dieser Abschnitte (32) zwischen einem Ende der Einschließung (6) und der im wesentlichen rohrförmigen starren Anschlussklemme (33) zum Anschluss an die unmittelbar aneinandergrenzende Feder (7) zwischengeschaltet ist.

30. Vorrichtung nach einem oder mehreren vorhergehenden Ansprüchen, **dadurch gekennzeichnet, dass** jeder Ankerabschnitt (32) eine im wesentlichen rohrförmige zylinderförmige Form mit im wesentlichen demselben Außendurchmesser wie den Einschließungen (6) aufweist und durch eine innere zylindrische Wand (132) und eine äußere zylindrische Wand (232) gebildet ist, die koaxial angeordnet sind und einen Raum dazwischen bestimmen, wobei wenigstens eine der beiden Wände (132, 232), bevorzugt die Außenwand (232) aus einem ausziehbaren oder ausdehnbaren Material, wie Gummi oder dergleichen besteht.

31. Vorrichtung nach einem oder mehreren vorhergehenden Ansprüchen, **dadurch gekennzeichnet, dass** die Verriegelung durch nach außen gerichtete radiale Dehnung der Außenwand (232) jedes Ankerabschnittes (32) erfolgt, die durch Einleiten einer Druckflüssigkeit in den leeren Raum zwischen den beiden Wänden (132, 232) des Abschnitts (32) erfolgt.

32. Vorrichtung nach einem oder mehreren vorhergehenden Ansprüchen, **dadurch gekennzeichnet, dass** mindestens eine Leitung (34), insbesondere eine einzige durchgehende Leitung (34) zur Versorgung von Druckflüssigkeit an die Anker- abschnitte (32) angeordnet ist, wobei diese Leitung (34) an ihrem äußeren Ende an eine Druckflüssigkeitsquelle angeschlossen ist, und sich innerhalb jedes Ankerabschnittes (32) parallel zu dessen Achse erstreckt und eine oder mehrere radiale Öffnungen (134) hat, die mit dem leeren Raum zwischen den beiden Wänden (132, 232) in Kommunikationsverbindung stehen.

33. Vorrichtung nach einem oder mehreren vorhergehenden Ansprüchen, **dadurch gekennzeichnet, dass** die Zufuhrleitung (34) durch Perforieren der Dicke der inneren undehnbaren rohrförmigen Wand (132) jedes Ankerabschnittes (32) ausgebildet ist.

34. Vorrichtung nach einem oder mehreren vorhergehenden Ansprüchen, **dadurch gekennzeichnet, dass** diese Zufuhrleitung (34) sich innerhalb jeder Einschließung (6), parallel zu deren Achse, erstreckt oder sich durch Perforieren der Dicke der Wand der Einschließung (6) ergibt.

35. Vorrichtung nach einem oder mehreren vorhergehenden Ansprüchen, **dadurch gekennzeichnet, dass** auf dem gleichen Niveau wie den Schrauben-Anschlussfedern (7), wobei diese Leitung (34) durch einen Teil eines flexiblen, insbesondere spiralförmigen Rohrs mit einem Radius bildet ist als die Federn selbst, um Verformungen der Federn (7) ohne mögliche Störung unterbringen zu können.

36. Vorrichtung nach einem oder mehreren vorhergehenden Ansprüchen, **dadurch gekennzeichnet, dass** eine oder mehrere der Einschließungen (6), insbesondere in einer Zwischenstellung ihrer Axialausdehnung, wenigstens ein Element (16) aufweisen, um einen oder mehrere Sensoren (12, 17, 18, 21) zu tragen.

37. Vorrichtung nach einem oder mehreren vorhergehenden Ansprüchen, wobei mindestens ein Erdbebensensor (17) in wenigstens einer der Einschließungen (6) vorhanden ist.

38. Vorrichtung nach einem oder mehreren vorhergehenden Ansprüchen, wobei mindestens ein Temperatursensor (21) in wenigstens einer der Einschließungen (6) vorhanden ist.

39. Vorrichtung nach einem oder mehreren vorhergehenden Ansprüchen, wobei mindestens ein Druck- oder piezometrischer Niveausensor (21) in wenigstens einer der Einschließungen (6) vorhanden ist.

40. Vorrichtung nach einem oder mehreren vorhergehenden Ansprüchen, wobei mindestens ein elektrischer Potentialmeter in wenigstens einer der Einschließungen (6) vorhanden ist.

41. Vorrichtung nach einem oder mehreren vorhergehenden Ansprüchen, wobei eine elektronische Karte (19) in wenigstens einer der Einschließungen (6) vorhanden ist, um die von den Sensoren (12, 17, 18, 21) Daten der entsprechenden Einschließung (6) zu sammeln und/oder örtlich zu bearbeiten.

42. Vorrichtung nach einem oder mehreren vorhergehenden Ansprüchen, **dadurch gekennzeichnet, dass** sie ein vieladriges Kabel (22) zur Übertragung der durch Sensoren (12, 17, 18, 21) gesammelten Daten enthält, das sich von der letzteren Einschließung (6) zur ersten Einschließung (6) der Reihenfolge von Einschließungen (6) erstreckt und der Reihe nach durch einen flüssigkeitsdichten Durchgang (31, 31') nach außen geführt wird.

43. Vorrichtung nach einem oder mehreren vorhergehenden Ansprüchen, **dadurch gekennzeichnet, dass** auf dem gleichen Niveau wie den Schrauben-Anschlussfedern (7), das vieladrige Übertragungskabel (22) mit einem geringeren Radius spiralförmig ist als die Federn, um deren perfekte Passform zur Vermeidung von Quetschgefahren zu gewährleisten.

44. Vorrichtung nach einem oder mehreren vorhergehenden Ansprüchen, **dadurch gekennzeichnet, dass** die Wicklung der Feder (7) mindestens ein Element (36) umfasst, das sich axial von einem Ende zum anderen Ende der Feder (7) erstreckt, wobei dieses Element (36) aus einem elastisch verformbaren Material besteht, um axial kompressibel/ausziehbar und verformbar zu werden, wobei mindestens eine Spiralnut (136) an der äußeren Oberfläche dieses Elements (36) vorgesehen ist, um die Zufuhrleitung (34) der Druckflüssigkeit und/oder das vieladrige Übertragungskabel (22) zu unterbringen.

45. Vorrichtung nach einem oder mehreren vorhergehenden Ansprüchen, **dadurch gekennzeichnet, dass** das vieladrige Übertragungskabel (22) diskontinuierlich ist und die Karten verbindet (19) zum Sammeln der von den Sensoren (12, 17, 18, 21) bereitgestellten Daten, die den Einschließungen (6) zugeordnet sind, wobei diese Datensammelkarten (19) Eingänge und Ausgänge für die Übertragungslinie, und einen Empfangsabschnitt enthalten, auf der Basis von Mehrkanal- oder Mehrprogramm-Protokollen oder Übertragungsbussen.

46. Vorrichtung nach einem oder mehreren vorhergehenden Ansprüchen, **dadurch gekennzeichnet, dass** der Temperatursensor (21) und/oder der Drucksensor mit der Außenseite ihrer jeweiligen Einschließungen (6) in Kommunikationsverbindung stehen können, wobei der empfindliche Teil dieser Sensoren (21) in einer äußeren Seitenausnehmung (26) oder in einem Hinterschnitt der Einschließung (6) untergebracht ist oder mit diesen in Verbindung steht, wobei er von der Innenseite der Einschließung (6) dicht abgeschlossen ist.

47. Vorrichtung nach einem oder mehreren vorhergehenden Ansprüchen, **dadurch gekennzeichnet, dass** die Umfangswand der Einschließung (6) eine Außenwand (126) mit Mikrovertiefungen an der Ausnehmung (26) zur Kommunikation zwischen den Sensoren (21) und dem Äußeren besitzt.

48. Vorrichtung nach einem oder mehreren vorhergehenden Ansprüchen, **dadurch gekennzeichnet, dass** die Ausnehmung (26), die durch Temperatur- und/oder Drucksensoren (21) benutzt wird, um mit dem Äußeren in Kommunikationsverbindung zu stehen, mit einem porösen Material gefüllt ist.

49. Vorrichtung nach einem oder mehreren vorhergehenden Ansprüchen, **dadurch gekennzeichnet, dass** eine lokale Steuereinheit (25) zur Sammlung von Daten angeordnet ist, die mit dem Übertragungskabel (22) verbunden ist und Kontrollen an die Datensammelkarten (19) und/oder die Sensoren (12, 17, 18, 21) überträgt.

50. Vorrichtung nach einem oder mehreren vorhergehenden Ansprüchen, **dadurch gekennzeichnet, dass** die lokale Einheit auch Radiomittel zum Empfang und zur Sendung von Kontrollen und Daten an einen und von einem Zentralüberwachungspunkt enthält.

51. Vorrichtung nach einem oder mehreren vorhergehenden Ansprüchen, **dadurch gekennzeichnet, dass** die lokale Einheit (25) bezogen auf die auszuführenden Funktionen programmierbar sein kann, wie Sensorbetätigungs- und/oder Lesezeiten (12, 17, 18, 21), Alarmschwellen oder dergleichen.

52. Vorrichtung nach einem oder mehreren vorhergehenden Ansprüchen, **dadurch gekennzeichnet, dass** die lokale Steuereinheit separat oder in einer ersten Einschließung (6) integriert sein kann.

53. Vorrichtung nach einem oder mehreren vorhergehenden Ansprüchen, **dadurch gekennzeichnet, dass** die flexiblen Fugen (3) Enden aufweisen, die zwischen zwei extremen Hubendepositionen (171) axial (72) gleiten, wobei diese gleitenden Enden mit elastischen der axialen Verlagerung entgegengesetzten Kräften beaufschlagt sind, was einen stoßdämpfenden Effekt gewährleistet.

54. Vorrichtung nach einem oder mehreren vorhergehenden Ansprüchen, **dadurch gekennzeichnet, dass** die ausdehnbaren Ankerelemente (32) auf das gleiche Niveau wie die starren rohrförmigen Einschließungen (6) gesetzt werden.

55. Vorrichtung nach Anspruch 54, **dadurch gekennzeichnet, dass** die Ankerelemente (32) rohrförmig sind und über der äußeren Fläche der starren Einschließungen (6) entlang wenigstens eines Teils deren axialen Erstreckung und/oder wenigstens eines Teils der umlaufenden Erstreckung dieser Außenfläche der starren rohrförmigen Einschließungen (6) liegen.

56. Vorrichtung nach Anspruch 54 oder 55, **dadurch gekennzeichnet, dass** die ausdehnbaren Ankerelemente (32) mit Druckflüssigkeit durch die Außenwand der starren rohrförmigen Einschließung (6) versorgt werden, nämlich mittels einer abgewinkelten Leitung, mit einer axialen Stange und einer radialen Stange, die in einem Endverschlusselement (306) der rohrförmigen Einschließung (6) ausgebildet ist und wobei diese Leitung mit ihrer axialen Stange mit einem Druckflüssigkeitsversorgungsrohr in und mit ihrer radialen Stange mit einer Durchführöffnung in Verbindung steht, die in der Außenfläche des starren rohrförmigen Elementes (6) ausgebildet ist, wobei diese Öffnung mit einer Versorgungsöffnung auf der aneinandergrenzenden Kontaktfläche des Ankerelements (32) gegen die Außenfläche der starren rohrförmigen Einschließung (6) zusammenfällt und abdichtbar verbunden ist.

57. Vorrichtung nach einem oder mehreren vorhergehenden Ansprüchen 54 bis 56, **dadurch gekennzeichnet, dass** jede Fuge (3) aus einer biegsamen Schraubenfeder (7) aufgebaut ist, die in einer flexiblen Hülse untergebracht ist, deren Enden von Anschlussklemmen (71) gebildet werden, die als gleitende Rollen für die jeweiligen axialen Schieber (72) wirken, jeder mit einer unrunden Welle, die sich durch ein komplementäres unrundes Loch in diesen Anschlussklemmen erstreckt und deren Ende innerhalb der jeweiligen Anschlussklemme (71) radial aufgeweitet (172) ist und auf zwei axial beabstandete radiale innere Anschlägen (71) anliegt, wobei ein Federelement, zum Beispiel eine Schraubenfeder zwischen dem radial aufgeweiteten Teil (172) des Schiebers (72) und den radialen inneren Anschlägen (171) der entsprechenden Anschlussklemme (71) angeordnet ist.

58. Vorrichtung nach einem oder mehreren vorhergehenden Ansprüchen 54 bis 57, **dadurch gekennzeichnet, dass** axiale Durchgangslöcher in den Wellen der axialen Schieber (72) der Fugen (3) ausgebildet sind.

59. Vorrichtung nach einem oder mehreren vorhergehenden Ansprüchen 54 bis 58, **dadurch gekennzeichnet, dass** die äußere, Enden der axialen Schieber (72) Mittel zur mechanischen Befestigung dieser an jeweiligen Modulen, d.h. starren rohrförmigen Einschließungen (6) aufweisen.

## Revendications

1. Appareil pour contrôler les paramètres géotechniques et structurels des sols (3), des roches et des structures en général, comprenant au moins un boîtier (6) logeant au moins un capteur (12, 17, 18, 21) pour au moins un paramètre, **caractérisé en ce qu'**il comprend au moins deux, de préférence une pluralité de boîtiers rigides (6), chacun pour loger au moins un type de capteur (12, 17, 18, 21), lesquels boîtiers rigides (6) sont raccordés par des éléments de raccordement non rigides (7),
dans lequel :
les boîtiers (6) sont agencés successivement les uns après les autres le long d'une ligne prédéterminée ou d'un axe prédéterminé, au moins un élément de raccordement non rigide (7) étant intercalé entre deux boîtiers (6) immédiatement adjacents,
les éléments de raccordement (7) permettent un déplacement axial relatif de deux boîtiers (6) adjacents vers et/ou à distance l'un de l'autre, et
un déplacement angulaire relatif de deux boîtiers (6) adjacents, **caractérisé en ce que** :
au moins deux boîtiers (6) adjacents sont raccordés par un tirant (13), au moins l'une des extrémités du tirant (13) étant dynamiquement raccordée à un capteur (12) qui détecte la position axiale relative des deux boîtiers (6) et qui est logé dans lesdits boîtiers (6) ;
au moins un capteur d'inclinaison (17) est prévu dans au moins l'un des boîtiers (6).

2. Appareil selon la revendication 1, **caractérisé en ce que** chacun des boîtiers (6) et/ou des éléments de raccordement non rigides (7) sont du type fermé, et imperméables au moins aux liquides extérieurs.

3. Appareil selon la revendication 1 ou 2, **caractérisé en ce que** les boîtiers (6) sont agencés de manière alternée par rapport aux éléments de raccordement (7).

4. Appareil selon une ou plusieurs des revendications précédentes, **caractérisé en ce que** les éléments de raccordement (7) entre les boîtiers (6) sont élastiques, et rétablissent la condition de positionnement de boîtier (6) mutuelle initiale, en référence à au moins un degré de liberté de mouvement ou à tous les degrés de liberté de mouvement des boîtiers (6) entre eux.

5. Appareil selon une ou plusieurs des revendications précédentes, **caractérisé en ce que** les éléments de raccordement non rigides comprennent des moyens élastiques (7) pour rétablir la condition de repos ou non sollicitée initiale de l'appareil.

6. Appareil selon une ou plusieurs des revendications précédentes, **caractérisé en ce que** les boîtiers (6) ont une forme tubulaire, de préférence cylindrique et en particulier ont des diamètres identiques et des extensions axiales identiques.

7. Appareil selon une ou plusieurs des revendications précédentes, **caractérisé en ce que** les éléments de raccordement (7) ont une forme essentiellement tubulaire et s'étendent le long des surfaces tubulaires, de préférence cylindriques ; en particulier ils peuvent être formés par des sections de tubes en caoutchouc renforcés avec une tresse d'acier, pour résister aux déviations angulaires des boîtiers (6), provoquées par les contraintes de traction et de compression axiales, sans leur communiquer de rotation.

8. Appareil selon la revendication 7, **caractérisé en ce que** les éléments de raccordement (7) sont des ressorts hélicoïdaux (7) agencés de manière coaxiale sur les boîtiers (6) tubulaires.

9. Appareil selon une ou plusieurs des revendications précédentes, **caractérisé en ce que** chaque ressort hélicoïdal (7) est fixé au niveau de ses deux extrémités opposées aux extrémités correspondantes des deux boîtiers (6) adjacents.

10. Appareil selon une ou plusieurs des revendications précédentes, **caractérisé en ce que** chaque ressort hélicoïdal (7) est recouvert hermétiquement vers l'extérieur par au moins un manchon flexible tubulaire, en particulier en forme de soufflet (11), de préférence réalisé à partir d'une matière plastique, de caoutchouc ou similaire et éventuellement renforcé par une tresse d'acier.

11. Appareil selon une ou plusieurs des revendications précédentes, **caractérisé en ce qu'**au moins deux boîtiers (6) adjacents sont raccordés par un tirant (13) ayant une longueur telle qu'il maintient le ressort hélicoïdal (7) dans une condition de charge de pression intermédiaire.

12. Appareil selon une ou plusieurs des revendications précédentes, **caractérisé en ce que** ledit capteur (12) est un aimant permanent qui peut coulisser dans deux directions vers le tirant (13), lequel aimant coopère avec un capteur d'induction pour déterminer le déplacement de l'aimant par l'induction magnétique.

13. Appareil selon une ou plusieurs des revendications précédentes, **caractérisé en ce que** ledit capteur (12) est équipé avec des moyens élastiques pour tirer l'aimant vers la compression du ressort de raccordement (7) immédiatement adjacent.

14. Appareil selon une ou plusieurs des revendications précédentes, **caractérisé en ce que** le tirant (13) a une longueur telle qu'il maintient l'aimant du boîtier (6) par rapport au capteur de position axiale (12) dans une position de signalisation intermédiaire lorsque l'appareil est dans une position de repos ou non sollicitée.

15. Appareil selon une ou plusieurs des revendications précédentes, **caractérisé en ce que** ledit tirant est un fil (13) ou une tige, en particulier gainé(e) (14), et s'étend sensiblement le long de l'axe central de l'appareil.

16. Appareil selon une ou plusieurs des revendications précédentes, **caractérisé en ce que** les boîtiers (6), les éléments de raccordement (7) et les tirants de raccordement (13) entre les boîtiers (6) forment un ensemble d'éléments pour transférer les contraintes de déplacement de sol (3) vers les capteurs de position, de pression, d'inclinaison et d'accélération (12, 17, 18, 21) ou des transducteurs ou similaires.

17. Appareil selon une ou plusieurs des revendications précédentes, **caractérisé en ce que**, à chacune de leurs extrémités opposées, les boîtiers (6) ont un élément de fermeture (8, 8') qui peut être inséré et fixé à l'intérieur de ces derniers, et agit en tant que borne permettant la fixation et/ou le passage des ressorts de raccordement (7), des manchons d'étanchéité (11), des tirants (13) et des un ou plusieurs capteurs de position (12).

18. Appareil selon une ou plusieurs des revendications précédentes, **caractérisé en ce qu'**au niveau de chaque extrémité opposée de chaque boîtier (6), on prévoit une borne (33) rigide sensiblement tubulaire pour coupler chaque extrémité du boîtier (6) aux extrémités correspondantes des deux ressorts (7) immédiatement adjacents.

19. Appareil selon une ou plusieurs des revendications précédentes, **caractérisé en ce qu'**au moins certains des éléments de raccordement (7) ont des moyens formant butée d'extension axiale (35).

20. Appareil selon une ou plusieurs des revendications précédentes, **caractérisé en ce qu'**au moins certains des éléments de raccordement (7) ont des moyens formant butée de compression axiale (35).

21. Appareil selon une ou plusieurs des revendications précédentes, **caractérisé en ce que** lesdits moyens formant butée d'extension ou de compression sont un ou plusieurs éléments allongés inextensibles (35), s'étendant chacun vers l'intérieur ou vers l'extérieur de chaque ressort (7), parallèle à leur axe, l'une des extrémités opposées (135) dudit élément inextensible (35) ayant une forme élargie et étant logée de manière coulissante à l'intérieur d'une chambre ayant deux butées de compression et d'extension opposées (133, 233), ladite chambre étant prévue à l'intérieur de l'élément de fermeture (8, 8') à l'extrémité d'un boîtier (6) adjacent ou à l'intérieur de la borne de couplage (33) rigide sensiblement tubulaire, alors que l'extrémité opposée de l'élément inextensible (53) est fixée au niveau de l'élément de fermeture (8, 8') ou au niveau de l'élément rigide (33) pour coupler le ressort (7) à l'autre boîtier (6).

22. Appareil selon une ou plusieurs des revendications précédentes, **caractérisé en ce que** chacune des extrémités opposées (135) de chaque élément inextensible (35) a une forme élargie et est logée de manière coulissante à l'intérieur d'une chambre ayant deux butées d'extension et de compression (133, 233) opposées, chacune desdites chambres étant prévues à l'intérieur de chaque élément de fermeture (8, 8') opposé au niveau de chaque extrémité de chaque boîtier (6) ou à l'intérieur des bornes de couplage (33) rigides sensiblement tubulaires.

23. Appareil selon une ou plusieurs des revendications précédentes, **caractérisé en ce que** chacun desdits éléments de butée inextensibles est un fil (35) ou une tige, de préférence réalisé à partir d'acier harmonique, lesdits éléments inextensibles (35) étant agencés le long de la circonférence externe du ressort (7).

24. Appareil selon une ou plusieurs des revendications précédentes, **caractérisé en ce que**, lorsque l'appareil est dans la condition au repos ou non sollicitée, les extrémités élargies (135) de chaque élément inextensible (35) sont dans une position de butée de compression d'un ressort (7).

25. Appareil selon une ou plusieurs des revendications précédentes, **caractérisé en ce que** l'on prévoit des moyens (32) pour fixer de manière amovible au moins une partie de l'appareil sur le sol (1), la roche ou la structure à contrôler.

26. Appareil selon une ou plusieurs des revendications précédentes, **caractérisé en ce que** lesdits moyens de fixation sont une ou plusieurs sections d'ancrage (32), lesquelles sections (32) sont agencées dans un ordre prédéterminé par rapport aux boîtiers (6) et aux ressorts de raccordement (7).

27. Appareil selon une ou plusieurs des revendications précédentes, **caractérisé en ce que** lesdites sections d'ancrage (32) sont du type fermé et imperméable au moins aux liquides extérieurs.

28. Appareil selon une ou plusieurs des revendications précédentes, **caractérisé en ce que** l'action de verrouillage est obtenue par l'expansion radiale des sections d'ancrage (32) à l'intérieur du trou (1) dans lequel l'appareil est logé.

29. Appareil selon une ou plusieurs des revendications précédentes, **caractérisé en ce qu'**une section d'ancrage (32) est respectivement prévue immédiatement en amont et immédiatement en aval de chaque boîtier (6), chacune desdites sections (32) étant intercalée entre une extrémité du boîtier (6) et la borne (33) rigide sensiblement tubulaire pour le raccordement au ressort (7) immédiatement adjacent.

30. Appareil selon une ou plusieurs des revendications précédentes, **caractérisé en ce que** chaque section d'ancrage (32) a une forme cylindrique sensiblement tubulaire, sensiblement avec le même diamètre externe que les boîtiers (6), et est formée par une paroi (132) cylindrique interne et une paroi (232) cylindrique externe qui sont agencées de manière coaxiale et définissent un espace entre elles, au moins l'une des deux parois (132, 232), de préférence la paroi externe (232), étant réalisée avec un matériau extensible ou expansible, comme le caoutchouc ou similaire.

31. Appareil selon une ou plusieurs des revendications précédentes, **caractérisé en ce que** l'action de verrouillage est obtenue par une expansion radiale vers l'extérieur de la paroi externe (232) de chaque section d'ancrage (32), qui est obtenue en introduisant un fluide sous pression, dans l'espace vide entre les deux parois (132, 232) de la section (32).

32. Appareil selon une ou plusieurs des revendications précédentes, **caractérisé en ce qu'**au moins un conduit (34), en particulier un seul conduit continu (34) est prévu pour amener le fluide sous pression aux sections d'ancrage (32), lequel conduit (34) est raccordé au niveau de son extrémité externe à une source de fluide sous pression, et s'étend à l'intérieur de chaque section d'ancrage (32), parallèle à son axe, et a une ou plusieurs ouvertures radiales (134) qui communiquent avec l'espace vide entre les deux parois (132, 232).

33. Appareil selon une ou plusieurs des revendications précédentes, **caractérisé en ce que** le conduit d'alimentation (34) est formé en perforant l'épaisseur de la paroi interne (132) tubulaire non expansible de chaque section d'ancrage (32).

34. Appareil selon une ou plusieurs des revendications précédentes, **caractérisé en ce que** ledit conduit d'alimentation (34) s'étend à l'intérieur de chaque boîtier (6), parallèle à son axe, ou est obtenu en perforant l'épaisseur de la paroi du boîtier (6).

35. Appareil selon une ou plusieurs des revendications précédentes, **caractérisé en ce que**, au niveau des ressorts de raccordement (7) hélicoïdaux, ledit conduit (34) est formé par une partie de tube flexible, en particulier en spirale avec un plus petit rayon que les ressorts eux-mêmes, pour pouvoir accepter les déformations des ressorts (7) sans aucune interférence éventuelle entre eux.

36. Appareil selon une ou plusieurs des revendications précédentes, **caractérisé en ce qu'**un ou plusieurs des boîtiers (6) contiennent, en particulier dans une position intermédiaire de leur extension axiale, au moins un élément (16) pour porter un ou plusieurs capteurs (12, 17, 18, 21).

37. Appareil selon une ou plusieurs des revendications précédentes, **caractérisé en ce qu'**au moins un capteur sismique (17) est prévu dans au moins l'un des boîtiers (6).

38. Appareil selon une ou plusieurs des revendications précédentes, **caractérisé en ce qu'**au moins un capteur de température (21) est prévu dans au moins l'un des boîtiers (6).

39. Appareil selon une ou plusieurs des revendications précédentes, **caractérisé en ce qu'**au moins un capteur de niveau de pression ou piézométrique (21) est prévu dans au moins l'un des boîtiers (6).

40. Appareil selon une ou plusieurs des revendications précédentes, **caractérisé en ce qu'**au moins un potentiomètre électrique est prévu dans au moins l'un des boîtiers (6).

41. Appareil selon une ou plusieurs des revendications précédentes, **caractérisé en ce qu'**une carte électronique (19) est prévue dans au moins l'un des boîtiers (6), pour collecter et/ou traiter localement les données fournies par les capteurs (12, 17, 18, 21) du boîtier (6) correspondant.

42. Appareil selon une ou plusieurs des revendications précédentes, **caractérisé en ce qu'**il comprend un câble multicolore (22) pour transmettre les données collectées par les capteurs (12, 17, 18, 21), qui s'étend à partir du dernier boîtier (6) jusqu'au premier boîtier (6) de la succession de boîtiers (6), et est ensuite amené à l'extérieur par un passage étanche au liquide (31, 31').

43. Appareil selon une ou plusieurs des revendications précédentes, **caractérisé en ce que**, au niveau des ressorts de raccordement (7) hélicoïdaux, le câble de transmission multicolore (22) est enroulé en spirale avec un rayon plus petit que les ressorts, pour garantir un parfait ajustement sans risque de pincement.

44. Appareil selon une ou plusieurs des revendications précédentes, **caractérisé en ce que** l'enroulement de ressort (7) contient au moins un élément (36) qui s'étend de manière axiale d'une extrémité à l'autre du ressort (7), lequel élément (36) est réalisé avec un matériau élastiquement déformable, pour être axialement compressible/extensible et déformable, au moins une rainure hélicoïdale (136) étant prévue sur la surface externe dudit élément (36), pour loger le conduit d'alimentation en fluide sous pression (34) et/ou le câble de transmission multicolore (22).

45. Appareil selon une ou plusieurs des revendications précédentes, **caractérisé en ce que** le câble de transmission multicolore (22) est discontinu et raccorde les cartes (19) pour collecter les données fournies par les capteurs (12, 17, 18, 21) associés aux boîtiers (6), lesdites cartes de collecte de données (19) ayant des entrées et des sorties pour la ligne de transmission, et une section de réception et de transmission basée sur des protocoles multicanaux ou multitâches ou des bus de transmission.

46. Appareil selon une ou plusieurs des revendications précédentes, **caractérisé en ce que** le capteur de température (21) et/ou le capteur de pression peuvent communiquer avec l'extérieur de leurs boîtiers (6) respectifs, la partie sensitive desdits capteurs (21) étant logée ou communiquant avec un évidement du côté externe (26) ou un dégagement du boîtier (6), tout en étant hermétiquement isolé de l'intérieur du boîtier (6).

47. Appareil selon une ou plusieurs des revendications précédentes, **caractérisé en ce que** la paroi périphérique du boîtier (6) a une paroi externe (126) avec des microfentes au niveau de l'évidement (26) pour la communication entre les capteurs (21) et l'extérieur.

48. Appareil selon une ou plusieurs des revendications précédentes, **caractérisé en ce que** l'évidement (26) utilisé par les capteurs de température et/ou de pression (21) pour communiquer avec l'extérieur est rempli avec un matériau poreux.

49. Appareil selon une ou plusieurs des revendications précédentes, **caractérisé en ce que** l'on prévoit une unité de commande de collecte de données locales (25) qui est raccordée au câble de transmission (22) et commande la transmission aux cartes de collecte de données (19) et/ou aux capteurs (12, 17, 18, 21).

50. Appareil selon une ou plusieurs des revendications précédentes, **caractérisé en ce que** l'unité locale a en outre des moyens pour la transmission/réception radio des commandes et des données vers/d'un emplacement de surveillance central.

51. Appareil selon une ou plusieurs des revendications précédentes, **caractérisé en ce que** l'unité locale (25) peut être programmable par rapport aux fonctions à accomplir, comme un temps d'actionnement et/ou de lecture de capteur (12, 17, 18, 21), des seuils d'alarme ou similaires.

52. Appareil selon une ou plusieurs des revendications précédentes, **caractérisé en ce que** l'unité de commande locale (25) peut être séparée ou intégrée dans un premier boîtier (6).

53. Appareil selon une ou plusieurs des revendications précédentes, **caractérisé en ce que** les joints flexibles (3) ont des extrémités qui coulissent (72) de manière axiale entre deux positions de fin de course extrêmes (171), lesquelles extrémités coulissantes sont soumises aux forces élastiques opposées au déplacement axial, fournissant un effet d'amortissement.

54. Appareil selon une ou plusieurs des revendications précédentes, **caractérisé en ce que** les éléments d'ancrage (32) expansibles sont placés de niveau avec les boîtiers (6) rigides tubulaires.

55. Appareil selon la revendication 54, **caractérisé en ce que** les éléments d'ancrage (32) ont une forme tubulaire et se trouvent sur la surface externe des boîtiers (6) rigides le long d'au moins une partie de leur extension axiale et/ou sur au moins une partie de l'extension circonférentielle de ladite surface externe des boîtiers (6) rigides tubulaires.

56. Appareil selon la revendication 54 ou 55, **caractérisé en ce que** les éléments d'ancrage (32) expansibles sont alimentés avec du fluide sous pression par la paroi externe du boîtier (6) rigide tubulaire, au moyen d'un conduit coudé ayant une tige axiale et une tige radiale, qui est formé dans un élément de fermeture d'extrémité (306) du boîtier (6) tubulaire, et lequel conduit communique, avec sa tige axiale, avec un tube d'alimentation en fluide sous pression et grâce à sa tige radiale, avec une ouverture formée dans la surface externe de l'élément (6) rigide tubulaire, laquelle ouverture coïncide et est raccordée de manière étanche avec une ouverture d'alimentation sur la surface de contact adjacente de l'élément d'ancrage (32) contre la surface externe du boîtier (6) rigide tubulaire.

57. Appareil selon une ou plusieurs des revendications 54 à 56, **caractérisé en ce que** chaque joint (3) est composé d'un ressort hélicoïdal (7) flexible, qui est logé dans un manchon flexible dont les extrémités sont formées par des bornes (71) servant de rouleaux coulissants pour des glissières axiales (72) respectives, chacune ayant un arbre non rond qui passe à travers un trou non rond complémentaire dans lesdites bornes (71) et dont l'extrémité à l'intérieur de la borne (71) respective est radialement élargie (172) et vient en butée contre deux butées internes radiales (171) espacées de manière axiale, un élément élastique, par exemple un ressort hélicoïdal, étant prévu entre la partie radialement élargie (172) de la glissière (72) et les butées internes radiales (171) de la borne (71) correspondante.

58. Appareil selon une ou plusieurs des revendications 54 à 57, **caractérisé en ce que** les trous traversants axiaux sont formés dans les arbres des glissières (72) axiales des joints (3).

59. Appareil selon une ou plusieurs des revendications 54 à 58, **caractérisé en ce que** les extrémités externes des glissières axiales (72) ont des moyens pour les fixer mécaniquement aux modules respectifs, c'est-à-dire les boîtiers (6) rigides tubulaires.
